# EUROPEAN PATENT APPLICATION

(11) **EP 4 653 037 A2**
(43) Date of publication of application: **26.11.2025**
(21) Application number: 25208352.2
(22) Date of filing: 25.02.2021
(51) Int. Cl.: A61M 25/00

(54) **EXPANDABLE SHEATH WITH EXTRUDED SEGMENTS**

(30) Priority: 27.02.2020 US 202062982546 P
(62) Divisional of application: 21713229.9
(71) Applicant: Edwards Lifesciences Corporation, Irvine, CA 92614 (US)
(72) Inventor: Bian, Baigui, IRVINE, CA, 92614 (US); Bulman, Erik, IRVINE, CA, 92614 (US); Geiser, Timothy A., IRVINE, CA, 92614 (US)
(74) Representative: Eisenführ Speiser

(57) **Abstract**

The expandable sheaths disclosed herein include an elastic outer tubular layer and a multisegmented inner tubular layer that includes at least two coextruded segments having different durometers and different coefficients of friction. The inner tubular layer further includes a thick wall portion integrally connected to a thin wall portion. The thin wall portion has a lower durometer than the thick wall portion. The thick wall portion has a first and second longitudinally extending end, and the thin wall portion extends between the first and second longitudinally extending ends. The elastic outer tubular layer and the inner tubular layer are radially movable between a non-expanded state, where the elastic outer tubular layer urges the first longitudinally extending end under the second longitudinally extending end, and an expanded state, where the first and second longitudinally extending ends of the inner tubular layer expand apart with the thin wall portion extending therebetween.

## Description

### RELATED APPLICATIONS

This application claims the benefit of U.S. Provisional Application No. 62/982,546, filed February 27, 2020, which is incorporated by reference in its entirety for all purposes.

### FIELD

The present application concerns embodiments of a sheath for use with catheter-based technologies for repairing and/or replacing heart valves, as well as for delivering an implant, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (e.g., the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath, and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes, as well as the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced.

However, delivery and/or removal of prosthetic devices and other material to or from a patient still poses a risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

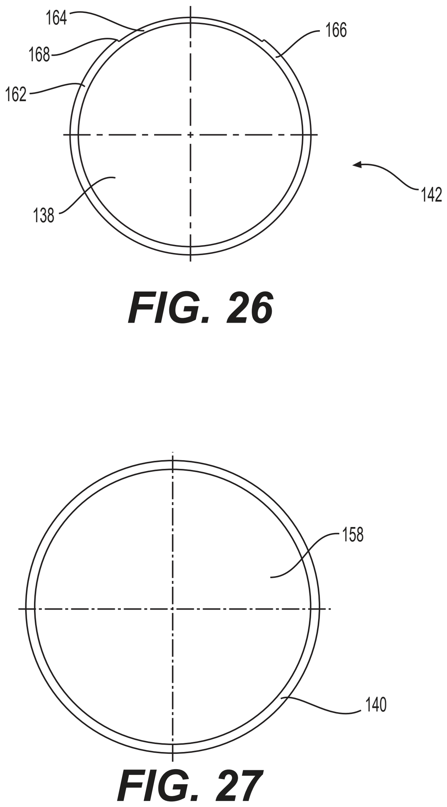

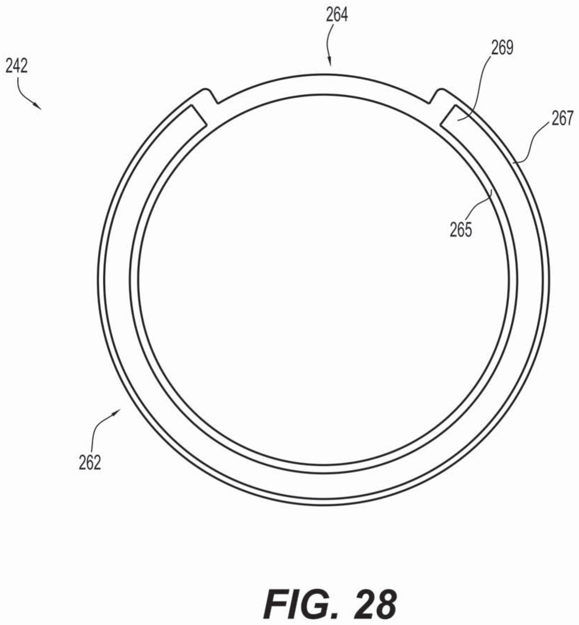

U.S. Patent No. 8,790,387, which is entitled EXPANDABLE SHEATH FOR INTRODUCING AN ENDOVASCULAR DELIVERY DEVICE INTO A BODY and is incorporated herein by reference (hereinafter, the '387 patent), discloses a sheath with a split outer polymeric tubular layer and an inner polymeric layer, for example in FIGS. 27A and 28. A portion of the inner polymeric layer extends through a gap created by the cut and can be compressed between the portions of the outer polymeric tubular layer. Upon expansion of the sheath, portions of the outer polymeric tubular layer have separated from one another, and the inner polymeric layer is expanded to a substantially cylindrical tube. Advantageously, the sheath disclosed in the '387 patent can temporarily expand for passage of implantable devices and then return to its starting diameter.

Despite the disclosure of the '387 patent, there remains a need for further improvements in introducer sheaths for endovascular systems used for implanting valves and other prosthetic devices.

### SUMMARY

Expandable sheaths are disclosed herein. The expandable sheaths include an elastic outer tubular layer and a multisegmented inner tubular layer. The multisegmented inner tubular layer includes at least two coextruded segments having different durometers and different coefficients of friction. The inner tubular layer further includes a thick wall portion integrally connected to a thin wall portion. The thin wall portion has a lower durometer than the thick wall portion. The thick wall portion has a first and second longitudinally extending end, and the thin wall portion extends between the first and second longitudinally extending ends of the thick wall portion. In some embodiments, the thick wall portion makes up greater than 50% of the circumference of a wall of the inner tubular layer. In some embodiments, the expandable sheath is an introducer sheath.

The elastic outer tubular layer and the inner tubular layer are radially movable between an expanded state and a non-expanded state. In the non-expanded state, the elastic outer tubular layer urges the first longitudinally extending end under the second longitudinally extending end of the inner tubular layer, such that the inner tubular layer has a fold in the unexpanded state. In the expanded state, the first and second longitudinally extending ends of the inner tubular layer expand apart, with the thin wall portion extending circumferentially therebetween. The outer elastic tubular layer urges the inner tubular layer back towards the non-expanded state.

As mentioned above, the multisegmented inner tubular layer includes at least two coextruded segments having different durometers and different coefficients of friction. The durometer and/or coefficient of friction of the inner tubular layer can vary radially through the thick wall portion. In some embodiments, the at least two coextruded segments can have different arc lengths extending in the circumferential direction. The at least two coextruded segments extend a portion of the length of the multisegmented inner tubular layer, or they can extend the full length of the multisegmented inner tubular layer.

In some embodiments, a radially outermost segment of the thick wall portion is formed of the same material as a radially innermost segment of the thick wall portion (such as, for example, HDPE). A radially intermediate segment of the thick wall portion can be included which has a higher durometer than the radially outermost segment and the radially innermost segment. In some embodiments, a radially intermediate segment of the thick wall portion is C-shaped in cross section and has an arc length that is less than the full arc length of the thick wall portion. For example, a radially outermost segment and a radially innermost segment can meet at longitudinally extending edges of the radially intermediate segment to fully envelop the radially intermediate segment.

In some embodiments, the thin wall portion is continuous with the material of the radially innermost segment and the radially outermost segment of the thick wall portion. In some embodiments, material of the thin wall portion can have a lower durometer than the material of the radially innermost segment and the radially outermost segment of the thick wall portion. In some embodiments, the thin wall portion is formed of a different coextruded segment than the radially innermost segment, the radially intermediate segment, and the radially outermost segment of the thick wall portion.

In some embodiments, the thin wall portion can include a first coextruded material, while the thick wall portion can include the first coextruded material as well as a second coextruded material positioned radially outward from the first coextruded material. The first coextruded material can form the radially innermost segment of the inner tubular layer and the second coextruded material can form the radially outermost segment of the inner tubular layer. In some embodiments, the first coextruded material can have a lower coefficient of friction than the second coextruded material.

Some embodiments of the expandable sheaths disclosed herein can include a coextruded tie layer. The coextruded tie layer can serve to adhere a first coextruded segment to a second coextruded segment. For example, the tie layer can adhere a radially innermost segment of the inner tubular layer to a radially outermost segment of the inner tubular layer.

In some embodiments, the outer tubular is seamless and prevents fluid leakage. The outer tubular layer can include a tapered proximal end, with the outer tubular layer thickening as it nears the proximal end of the sheath. In some embodiments, the tapered proximal end widens in a gradual manner to create a curved outer surface. The outer diameter of the outer tubular layer can increase nearing the proximal end of the sheath while the inner diameter of the outer tubular layer stays constant or changes by a value of less than 10% nearing the proximal end of the sheath. The outer tubular layer can include at least one longitudinally extending reinforcement formed of a higher durometer material than the material immediately adjacent to the reinforcement.

In some embodiments, the sheath is sized to accommodate the delivery of a heart valve. The outer diameter of the outer tubular layer can be, for example, from 0.22 inches to 0.30 inches. In some embodiments, the configuration of the inner tubular layer changes moving longitudinally such that a distal tip of the expandable sheath has a distinct configuration as compared to a longitudinally central shaft of the expandable sheath. In some embodiments, at least one surface of the sheath comprises a hydrophilic coating.

Delivery catheter assemblies that include the sheaths described above are also disclosed herein. In addition to an expandable sheath, a delivery catheter assembly can include a proximal region having a hub and a hemostasis valve. The sheath can be coupled to the hub, extending distally therefrom. In some embodiments, a tapered proximal end of the sheath widens as it extends toward the hub, and the tapered proximal end of the sheath is coupled to the hub. The sheath can further be fluidically coupled to the hemostasis valve, which can, in some embodiments, be housed within the hub. The proximal region of the delivery catheter assembly can further include a handle, and the handle can include an infusion port.

The delivery catheter assemblies can further include a guide catheter slidably positionable within the expandable sheath. In some embodiments, the guide catheter is steerable. The delivery catheter assemblies can further include a balloon catheter positionable within the guide catheter. A distal region of the balloon catheter includes an inflatable balloon, and, in some embodiments, a nose cone positioned distally from the inflatable balloon. An implantable device can be included, the implantable device being configured to be coupled to the inflatable balloon. In some embodiments, the implantable device is a heart valve. A capsule can be included, the capsule being configured to extend over the implantable device.

Methods of inserting an implantable device using the sheaths and delivery catheter assemblies described above are also disclosed herein. The methods can include inserting an expandable sheath at least partially into the blood vessel of the patient, advancing an implantable device through the inner tubular layer of the sheath, locally expanding the inner tubular layer from the compressed condition to the locally expanded condition using the outwardly directed radial force of the implant, and locally contracting the inner tubular layer from the locally expanded condition at least partially back to the compressed condition using inwardly directed radial force of the outer elastic tubular layer. In some embodiments, locally expanding further comprises moving the first and second longitudinally extending ends towards and then away from each other to reach the locally expanded condition. In some embodiments, locally contracting further comprises moving the first and second longitudinally extending ends toward and then away from each other to at least partially reach the compressed condition. In some embodiments, advancing an implantable device further comprises sliding a guide catheter through the expandable sheath with an implantable device coupled thereon.

Methods of positioning an implantable device within the vasculature of a patient using the devices and methods described above are also disclosed herein. The methods of positioning the implantable device further include advancing the implantable device distally beyond a distal tip of the sheath, positioning the implantable device within the vasculature of the patient, and removing the sheath from the blood vessel of the patient. The methods can further include expanding the implantable device (such as, but not limited to, a heart valve) within the vasculature of the patient. In some embodiments, expanding the implantable device within the vasculature of the patient can include inflating a balloon to apply a radially outward force on an inner surface of the implantable device. Some methods of positioning an implantable device can further include removing a capsule from the outer surface of the implantable device.

### DESCRIPTION OF DRAWINGS

FIG. 1 is an exploded side view of a delivery catheter assembly;
FIG. 2 is a cross-sectional view of a sheath of one embodiment of the present invention;
FIG. 3 is a partial exploded view of the sheath of FIG. 2;
FIG. 4 is an enlarged view of a distal end of the sheath of FIG. 2;
FIG. 5 is an enlarged view of a proximal end of the sheath of FIG. 2;
FIG. 6A is an enlarged view of a sheath of another embodiment with a capsule passing therethrough;
FIG. 6B is a cross sectional view of the sheath of FIG. 6A;
FIG. 7 is a cross sectional view of a sheath of another embodiment;
FIG. 8A is a schematic of another implementation of a delivery sheath with increasing elasticity approaching the distal end region;
FIGS. 8B-8D are cross sectional schematics of the delivery sheath implementation shown in FIG. 8A;
FIG. 9A is a schematic of another implementation of a delivery sheath with increasing elasticity approaching the distal end region;
FIGS. 9B-9D are cross sectional schematics of the delivery sheath implementation shown in FIG. 9A;
FIG. 10A is a schematic of another implementation of a delivery sheath with increasing elasticity approaching the distal end region;
FIGS. 10B-10D are cross sectional schematics of the delivery sheath implementation shown in FIG. 10A;
FIG. 11A is a schematic of another implementation of a delivery sheath with increasing elasticity approaching the distal end region;
FIGS. 11B-11D are cross sectional schematics of the delivery sheath implementation shown in FIG. 11A;
FIG. 12A is a schematic of another implementation of a delivery sheath with increasing elasticity approaching the distal end region.
FIGS. 12B-12D are cross sectional schematics of the delivery sheath implementation shown in FIG. 12A;
FIG. 13 is a schematic of assembly of two sheaths into a combination sheath of another embodiment of the present invention;
FIGS. 14-16 are cross-sections of embodiments sheaths having expandable thinned wall sections;
FIGS. 17-19 are cross-sections of embodiments of sheaths having wires or strips reinforcing expandable walled tubes;
FIG. 20 is a partial perspective view of a stent for an end of a sheath of another embodiment of the present invention; and
FIGS. 21-23 are perspective views of an embodiment of a stiff wall structure of a sheath having a distal stent portion progressively opening to increase its lumen diameter.
FIG. 24 shows a cross-section of inner and outer tubular layers of an additional sheath embodiment, in a non-expanded state.
FIG. 25 shows a perspective cross-sectional view of the sheath embodiment of FIG. 24, in a non-expanded state.
FIG. 26 shows a cross-sectional view of an example inner tubular layer of the sheath embodiment of FIGS. 24-25.
FIG. 27 shows a cross-sectional view of an example outer tubular layer of the sheath embodiment of FIGS. 24-25.
FIG. 28 shows a cross-sectional view of an example inner tubular layer of a sheath.
FIG. 29 shows a cross-sectional view of an example inner tubular layer of a sheath.
FIG. 30 shows a cross-sectional view of an example inner tubular layer of a sheath.
FIG. 31 shows a perspective view of a proximal end of an example outer tubular layer of a sheath.
FIG. 32 shows a cross-sectional view of an embodiment of a proximal end of an outer tubular layer of a sheath.
FIG. 33 shows a cross-sectional view of another embodiment of a proximal end of an outer tubular layer of a sheath.

### DETAILED DESCRIPTION

The following description of certain examples of the inventive concepts should not be used to limit the scope of the claims. Other examples, features, aspects, embodiments, and advantages will become apparent to those skilled in the art from the following description. As will be realized, the device and/or methods are capable of other different and obvious aspects, all without departing from the spirit of the inventive concepts. Accordingly, the drawings and descriptions should be regarded as illustrative in nature and not restrictive.

For purposes of this description, certain aspects, advantages, and novel features of the embodiments of this disclosure are described herein. The described methods, systems, and apparatus should not be construed as limiting in any way. Instead, the present disclosure is directed toward all novel and nonobvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present or problems be solved.

Features, integers, characteristics, compounds, chemical moieties, or groups described in conjunction with a particular aspect, embodiment or example of the invention are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract, and drawings), or to any novel one, or any novel combination, of the steps of any method or process so disclosed.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

As used herein, the term "substantially constant" means the first measurement differs from the second measurement by a value less than about 10 percent. In certain embodiments, the first measurement differs from the second measurement by less than 8 percent, less than 7 percent, less than 5 percent, less than 3 percent, less than 2 percent, or less than 1 percent.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal aspect. "Such as" is not used in a restrictive sense, but for explanatory purposes.

Disclosed herein is an expandable introducer sheath for passage of implant delivery catheters, such as catheters for delivery of prosthetic heart valves. The expandable sheath can minimize trauma to the vessel by allowing for temporary expansion of a portion of the expandable sheath to accommodate the delivery catheter, followed by a return to the original diameter once the implant passes through. Generally, disclosed herein, are various embodiments balancing the amounts, shapes and positions of various stiff and elastic structures in the sheath to selectively program the expandability and buckling stiffness of the sheath. The expandable sheath can include, for example, an expandable tubular layer that includes alternating stiff and elastic wall portions of a single radial thickness. The combination of stiff and elastic wall portions allow for torque and push strength to advance the expandable sheath while at the same time accommodating temporary expansion. The expandable sheath can also be reinforced with a tubular layer of braided fibers or a stent structure for additional strength. Other embodiments include selective use of slots or gaps at the distal end of a stiff wall portion to enhance expandability and distribute strain.

Disclosed herein are elongate delivery sheaths that are particularly suitable for delivery of implants in the form of implantable heart valves, such as balloon-expandable implantable heart valves. Balloon-expandable implantable heart valves are well-known and will not be described in detail here. An example of such an implantable heart valve is described in U.S. Patent No. 5,411,552, and also in U.S. Patent Application Publication No. 2012/0123529, both of which are hereby incorporated by reference. The elongate delivery sheaths disclosed herein may also be used to deliver other types of implantable devices, such as self-expanding implantable heart valves, stents or filters. The terms "implant" and "implantable" as used herein are broadly defined to mean anything - prosthetic or not - that is delivered to a site within a body. A diagnostic device, for example, may be an implantable.

The term "tube" or "tubular" as used herein is not meant to limit shapes to circular cross-sections. Instead, tube or tubular can refer to any elongate structure with a closed-cross section and lumen extending axially therethrough. A tube can also have some selectively located slits or openings therein - although it still will provide enough of a closed structure to contain other components within its lumen(s).

Expandable sheaths are described in U.S. Patent Nos. 9,987,134 and 10,327,896, and in U.S. Patent Application Publication No. 2018/0368979. Each of these documents is incorporated by reference in its entirety. The sheaths disclosed herein describe additional developments and advantages in expandable sheath technology.

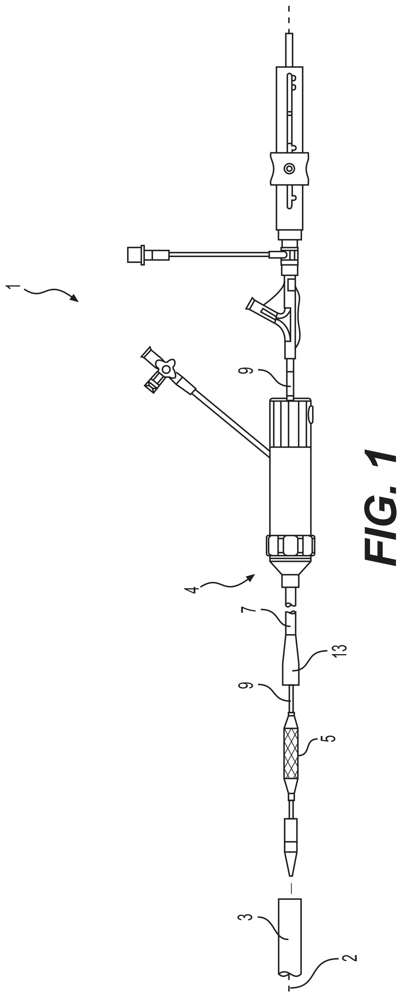

FIG. 1 illustrates a delivery catheter assembly 1 including an elongate, expandable delivery sheath 3 with a lumen to guide passage of an implant delivery catheter supporting a prosthetic implant 5, such as a prosthetic heart valve. At a proximal end the sheath 3 includes a hemostasis valve that prevents leakage of pressurized blood and a hub 4 for connecting with sheath 3. The delivery catheter assembly 1 can include a steerable guide catheter 7 (also referred to as a flex catheter) and a balloon catheter 9 extending through the guide catheter 7. The delivery catheter assembly 1 can also include a capsule 13 which has an enlarged diameter to hold the implant 5 mounted on the balloon of the balloon catheter 9.

Generally, during use, the sheath 3 is passed through the skin of patient (usually over a guidewire) such that the distal end region of the sheath 3 is inserted into a vessel, such as a femoral artery, and then advanced to a procedure site - such as over the aortic arch to a native aortic heart valve. The nose of the balloon catheter and capsule 13 is inserted through the hemostasis valve at the proximal end of the sheath 3. The steerable guide catheter 7 is used to advance the nose of the balloon catheter 9 and capsule 13 through to and out of the end of the sheath 3. The implant 5 is then advanced out of the capsule 13 and expanded into the native heart valve, such as by balloon inflation or by self-expansion.

The implementations of the delivery sheath shown herein can provide access for other implants and delivery devices needing transient expansion to facilitate passage of the implants or portions of the delivery devices. For example, in some implementations, the delivery sheath can be used to deliver oversized balloon catheters for angioplasty procedures. The term "implant" as used herein need not be a permanent implant - for example the balloon is an implant temporarily - but could be any device delivered into the body for a procedure.

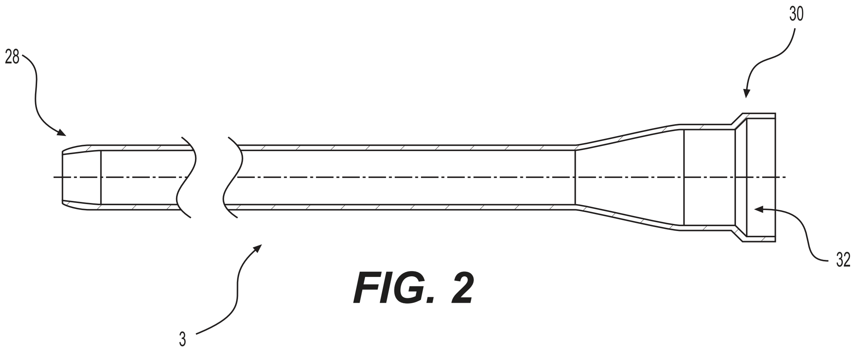

FIGS. 2-5 show one embodiment of sheath 3 including a wall structure having a tip 28 on its distal end and a tapered portion on its proximal end 30 and defining a lumen 32 extending therebetween. The wall structure includes an outer elastic layer 20, an intermediate mesh layer 22, a mixed expandable layer 24 and an inner lubricious low-friction liner or layer 26. Generally, the tapered proximal end 30 is sized and shaped to accept a distal male end of a hub structure containing, among other things, a hemostasis valve to mediate leakage during insertion of delivery catheters through the lumen 32 of the delivery sheath 3. The sheath 3 can be sized for delivery of prosthetic implants in the form, for example, of stent-mounted soft-tissue heart valves. For such an application, the sheath can have an outside diameter 0.260 inches and an inside diameter of 0.215 inches. Those diameters can vary with the size of the implant and/or the type of implant or other application.

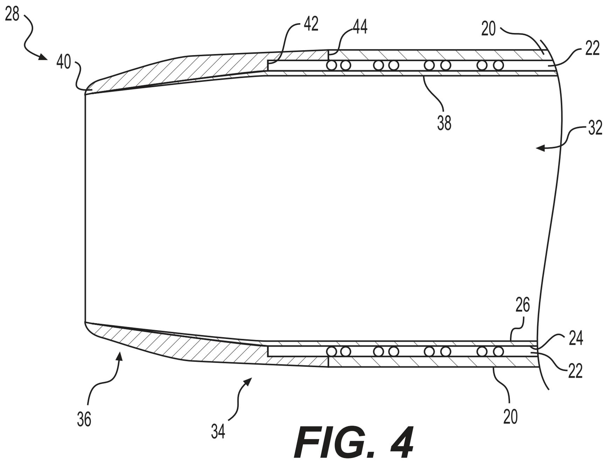

As shown in FIG. 4, the distal tip 28, which has a tapering cylindrical construction, has a proximal taper 34, a distal taper 36, an inner surface 38 and a rounded leading edge 40. The proximal taper 34 has a relatively slight angle with respect to the parallel outer walls of the outer elastic layer 20. Generally, the tip has an outside diameter of about 0.25 inches at the distal end of the proximal taper and an outside diameter of about 0.26 inches at the proximal end of the proximal taper 34. The distal taper 36 has a higher angle increasing to about 20 degrees. The distal taper 36 has a length of approximately 0.060 inches. The leading edge 40 has a rounded radius of about 0.01 inches. The outermost diameter of the leading edge is 0.206 inches and the inner most diameter of 0.187 inches.

The inner surface 38 supports a progressively thinning, distally tapering portion of the mixed expandable layer 24 and inner lubricious layer 26 - with the layers getting thinner in the distal direction. Together the inner surface and distally tapering portion of the layers 24, 26 define a distal portion of the lumen 32 through which the implant 5 and capsule 13 can exit.

At its proximal end the distal tip 28 includes an inner annular surface 42 and an outer annular surface 44. The inner annular surface is recessed within the proximal end of the distal tip 28 and the outer annular surface is on the proximal-most edge of the distal tip 28. The inner annular surface 42 is configured to receive and abut a distal edge of the mesh layer 22 and the outer annular surface 44 is configured to abut the distal edge of the outer elastic layer 20.

When assembled to the distal end of the layers 20, 22, 24 and 26 the distal tip 28 - which is constructed of a relatively smooth, rigid material - provides support for advancement of the distal end of the sheath 3. The tapers and rounded outer edges minimize trauma when advancing through body lumens. Also, the distal tip 28 helps to maintain the end diameter of the sheath 3 after passage of the implant 5 and capsule 13.

The outer layer 20 has a tubular shape and is preferably constructed of a soft elastomeric material, such as a polyether block amide (PEBAX) material or polyurethane (NEUSoft), so as to easily expand in response to forces and return to its original dimensions. Also, the elastomeric properties urge the more inner layers to contract back to their original shapes. The outer layer can have an outer diameter of from about 0.22 inches to about 0.30 inches (including about 0.22 inches, about 0.23 inches, about 0.24 inches, about 0.25 inches, about 0.26 inches, about 0.27 inches, about 0.28 inches, about 0.29 inches, and about 0.30 inches) and is the largest diameter of the layers making up the sheath 3. The outer layer 20 extends around and laminated onto the mesh layer 22 extending through its lumen.

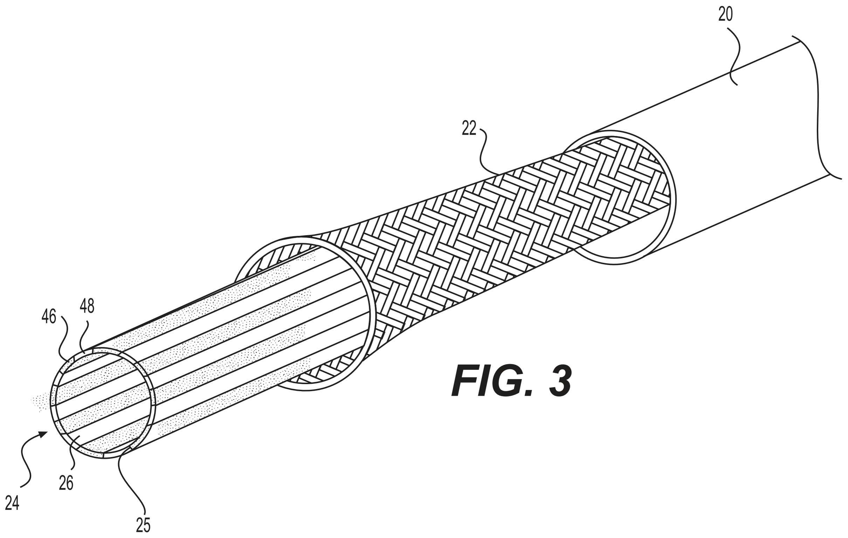

The mesh layer 22 is preferably formed of a textile that is comprised of less-elastic components that obtain flexibility and some push stiffness from woven or knit construction. For example, the mesh layer can be constructed of a PET (polyethylene terephthalate) rope or thread material that is woven into a flexible mesh or a sleeve or tube with porous openings to promote expansion and flexibility. The mesh layer 22 can be formed as a plurality of braided fibers. FIG. 3, for example, shows the tubular shape of one embodiment of the mesh layer 22 wherein one group of threads extends perpendicular to another group of threads. Wires or metal could also be used to construct the mesh layer 22, such as woven superelastic nitinol wires with high elastic strain limits.

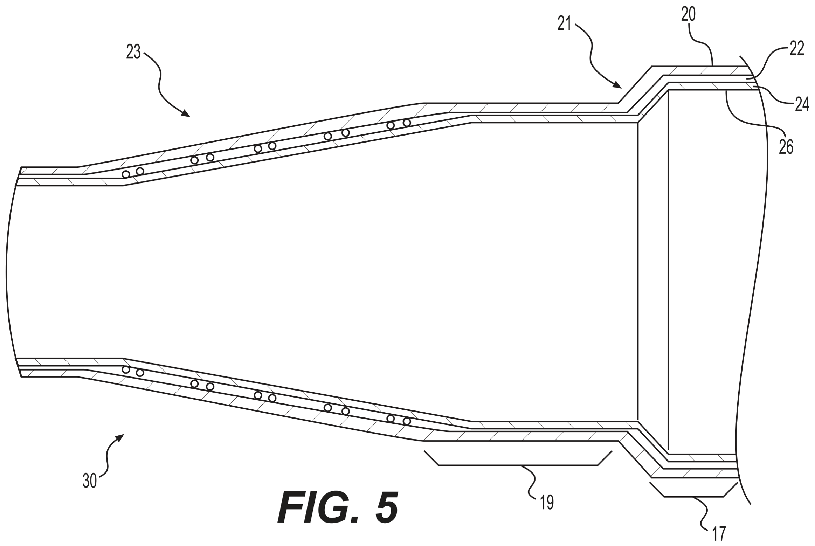

FIG. 5 shows a cross section of the tapered proximal end of sheath 3. Like the distal end, the proximal end includes an outer elastic layer 20, a middle mesh layer 22, a mixed expandable layer 24 and an inner lubricious liner or layer 26. The most proximal region has a first annular portion 17 that is wider than the remainder of sheath 3. The layers 20, 22, 24, and 26 narrow sharply moving distally from the first annular portion of the proximal end 30, forming shoulder 21. The shoulder 21 and first annular portion 17 are configured to connect to the hub 4 of the delivery system 1. Moving distally from the shoulder 21, the layers extend distally to form a second annular portion 19. The walls of the first and second annular portions 17, 19 extend substantially parallel to the longitudinal axis 2 of the sheath 3, and the second annular portion 19 extends a greater distance than the first annular portion 17. Moving distally from the second annular portion 19, the layers 20, 22, 24, and 26 narrow again to form a taper 23. Taper 23 makes a smaller angle with the longitudinal axis 2 than shoulder 21. Taper 23 also extends a greater distance along the longitudinal axis 2 than shoulder 21.

Referring again to FIG. 3, the mixed, expandable layer 24 is constructed of a mixture of alternating full-thickness portions, including soft portions 46 and hard portions 48. The soft portions 46 are constructed of elastomer material - such as materials similar to the outer layer 20 - that provide elasticity to the expandable layer 24. The hard portions 48 are constructed of a relatively stiff material and thus provide some columnar stability for advancing the sheath 3 against resistance of a body lumen. The number and spacing of the portions 46, 48 can be adjusted per application. Greater amounts or dimensions of stiff portions 48 can be included for more stiffness. Greater number or dimensions of soft/elastomeric portions 46 can be included for improved expandability and flexibility. TECOFLEX, an aliphatic polyether polyurethane, is one material that can be used for the stiff portions 48. In some embodiments, Nylon, or Nylon 12, can be used for the stiff portions 48.

The portions have a radial thickness from the inside to outside diameter that is equal about the circumference of the layer 24. Said another way, the wall thickness of layer 24 is consistent when viewed at a transverse cross section (a cross section perpendicular to the longitudinal axis of the layer 24). Also, each of the portions includes a pair of edges 25 between the hard and soft portions that extend between the inner and outer surfaces of the layer 24. The pair of edges can also extend longitudinally, in parallel to the long axis of the sheath 3. The soft/elastomeric portions 46 alternate with the hard portions 48 in arc-segments, their edges in abutting attachment, to form the tubular structure (with a consistent or constant wall thickness) of the mixed expandable layer 24. The hard and soft arc-segments can be equally sized, or they can vary in size as shown in FIG. 3.

The inner lubricious layer 26 coats or is adhered on inside surfaces of the expandable layer 24. The layer 26 is preferably a low-friction layer (such as PTFE) and can include a tie-layer attaching the lubricious material to the expandable layer 24. Advantageously, the composite of three layers - including an elastic outer layer, mesh layer and alternating hard/elastomeric layer and inner lubricious liner can provide a good balance of stiffness, expansion/recovery and low resistance to passage of implants.

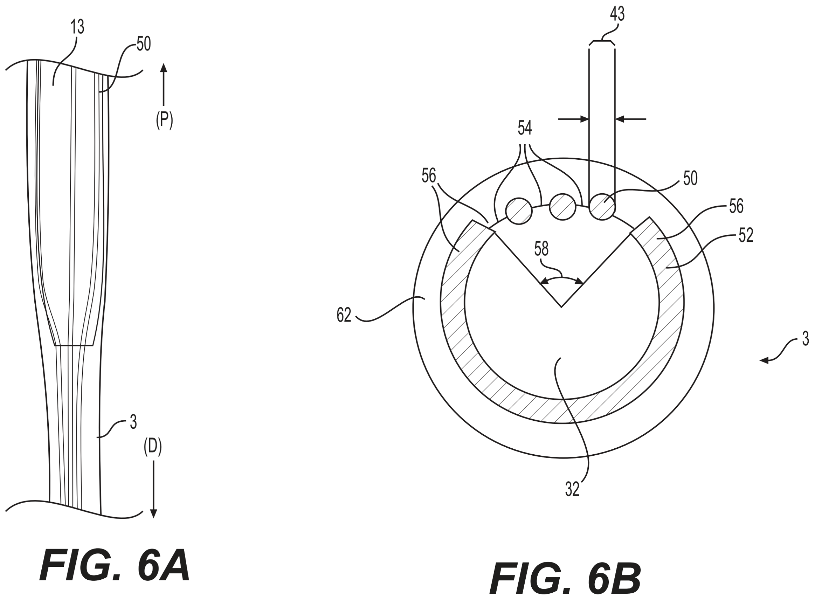

FIGS. 6A shows the delivery sheath 3 of another embodiment of the present invention with the capsule 13 carrying a stent-mounted heart valve or other prosthetic implant 5 passing through the sheath's lumen 32. (For example, the implant can be a 29 mm stent-mounted prosthetic heart valve.) The capsule 13 is passing in a proximal to distal direction. As used herein, "distal" (marked "D" in FIG. 6A), means towards the implantation site, and "proximal" (marked "P" in FIG. 6A) means away from the implantation site. In FIG. 6A, the delivery sheath 3 is depicted as transparent to permit illustration of capsule 13. However, a delivery sheath incorporating radiopaque material will actually cause the sheath 3 to opaque. Generally, the sheath of FIGS. 6A and 6B exhibits the ability to temporarily expand for passage of an oversized implant 5 and then return back to its normal diameter afterwards. Also, the sheath 3 can include multiple rods 50, that can be seen through the sheath, and that facilitate lower friction passage of the capsule 13.

FIG. 6B shows a cross section of the delivery sheath 3 including a stiff wall portion 52, an elastic wall portion 54 and the rods 50. The stiff wall portion 52 has a partial circular, or arc-shaped, or C-shaped cross-section with a consistent wall thickness within the cross-section. The C-shape of the stiff wall portion has a pair of edges 56 that extend between the inner and outer surfaces of the stiff wall portion 52. Perpendicular to the cross-section, the two edges extend generally along the length of the stiff wall portion 52 and in the direction of, and parallel to, the elongate axis of the delivery sheath 3.

The elastic wall portion 54 extends between the free edges 56 of the stiff wall portion 52 to define an expandable tubular layer and close the lumen 32 of the sheath 3. As shown in FIG. 6B, the elastic wall portion generally has a shorter arc-length than the stiff wall portion 52 and is positioned further away radially from the axis of the sheath 3 than the inside surface of the stiff wall portion 52. This additional radial clearance provides room for the three rods 50 to extend into the lumen 32. The elastic wall portion 54 can comprise an angle 58 of at least 20 degrees, or as much as 45 to 90 degrees of the cross-section of the sheath 3. The combination and proportions of the elastic and stiff wall portions 54, 52 provide for the temporary expansion and return of the lumen diameter 32 during passage of the implant 5.

The elastic wall portion 54 can be part of an outer elastic tubular layer 62 that externally encapsulates the stiff wall portion 52 in a seamless elastomeric layer. In this manner, the elastic tubular layer 62 helps to seal off the lumen 32 and to urge the C-shaped stiff wall portion 52 back to its original diameter when no longer under pressure from a passing implant. Although the sheath of FIGS. 6A and 6B can have a range of dimensions to suit different applications, the stiff wall portion 52 can, for prosthetic valve delivery purposes, range from 0.002 inches to 0.020 inches in thickness, including about 0.015 inches. The outer portion of the elastic tubular layer 62 adds about another 0.002 inches to 0.020 inches, and in particular about 0.005 inches. In one application, then, the total thickness of the sheath 3 wall can be about 0.020 inches. The unexpanded lumen 32 can have a diameter from 0.050 to 0.250 inches, such as 0.156 inches.

FIG. 6B shows three of the rods 50 embedded into the elastic wall portion 54 and extending into the lumen 32 of the sheath 3. The rods 50 are elongate structures with extruded cross sections - such as a cylindrical shape with a circular cross-section - that extend along the longitudinal axis of the sheath 3. The rods 50 of FIG. 6B are equally spaced from each other in a circumferential direction between the edges 56 of the C-shaped stiff wall portion 52. Advantageously, the spacing of the rods 50 can increase, as shown in FIG. 6A, during passage of the capsule 13 with stretching of the elastic wall portion 54. Thus the rods can provide some additional stiffness and reduce the surface area and friction that would otherwise be present between the elastic wall portion and the passing implant or capsule without much impact on the expandability of the sheath. As can be seen, at least about half of the cross-section of the rods 50 extends into the lumen 32.

The C-shaped stiff wall portion 52 can be comprised of a range of stiff materials, such as a high-density polyethylene or nylon which provides buckle resistance, pushability, torqueability and a relatively stiff body for the sheath 3. The combination of the elastomeric soft portion 46 helps to mediate kinks of the sheath and to bias against the opening tendency of the stiff wall portion 52. A proximal end of the expandable tubular layer including the wall portions 52, 54 and the outer elastic tubular layer 62 can be tapered to provide for hub attachment. Also, a tip could be constructed from the same elastomeric material as the wall portion 54. The tip could include radiopaque properties and be heat fused to the outer tubular layer 62. Manufacture is fairly easy since the components of the sheath 3 can be co-extruded in a single operation.

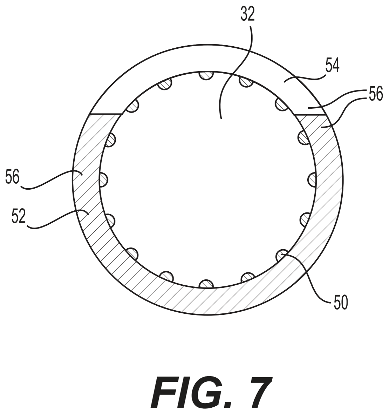

FIG. 7 shows another embodiment of sheath 3 including wall portions 52, 54 and rods 50 similar to the sheath 3 in FIGS. 6A and 6B. In this embodiment, however, the edges 56 of the stiff wall portion 52 are oriented to be within a common plane. The elastic wall portion 54 also has a thickness matched to the stiff wall portion 52, as opposed to having the encapsulating outer elastic tubular layer 62. The elastic wall portion 54 also takes up a larger angle 58 than the embodiment shown in FIGS. 6A and 6B.

The sheath 3 also includes a larger number of rods 50 which are equally spaced circumferentially about the entire lumen 32 and increase the overall stiffness of the sheath. The rods 50 are connected to the inside surfaces of both the stiff wall portion 52 and the elastic wall portion 54. The rods 50 have a semi-circular extruded cross-section. The additional rods 50 can further reduce contact area and the associated friction. The rods 50 can be comprised of stiff, relatively lubricious material to further facilitate sliding.

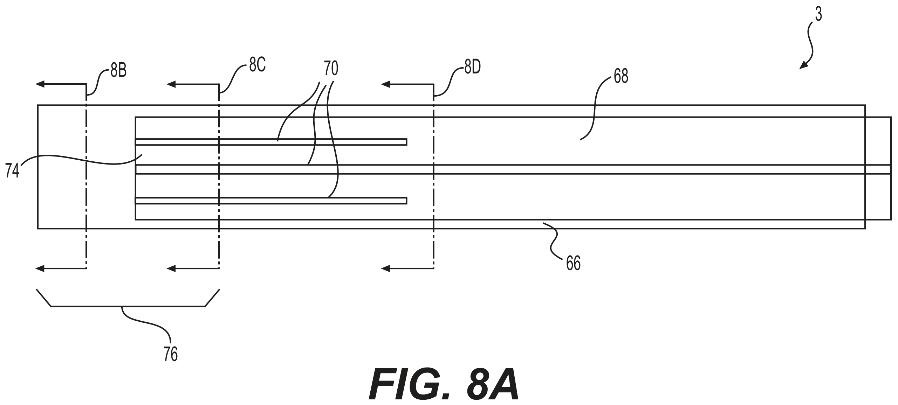

FIGS. 8A-8D show embodiments wherein the sheath 3 includes an elastic tubular layer 66 having covering one or more stiff wall portions 68. The elastic tubular layer 66 can be a seamless outer layer that guards against blood or fluid leakage. The stiff wall portions define one or more gaps 70. Generally, the cumulative circumferential amount of the cross-section taken up by the gaps 70 is proportional to the resistance to expansion of the sheath 3 at that particular longitudinal position. FIGS. 8A-8D, for example, show the cumulative amount of the gaps 70 increasing distally so that the amount of compression exerted on the implant drops in the distal direction. This can be advantageous as the friction and/or other resistance to advancement of the capsule 13 within the sheath can increase with increase in distance of travel - the drop in expansion resistance can offset somewhat the increased push resistance.

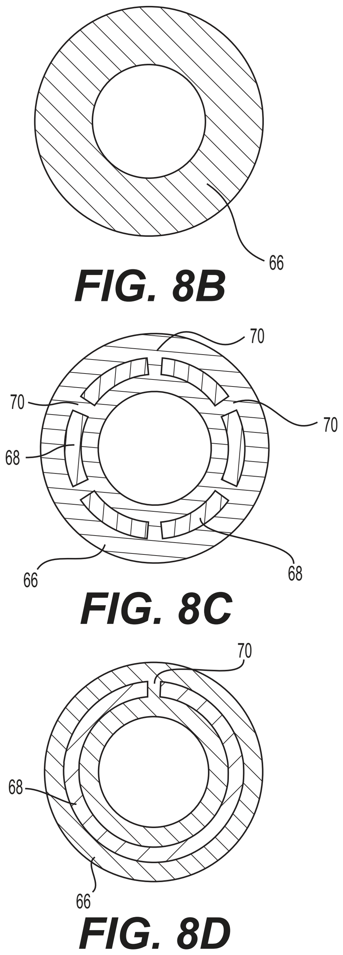

The cross-section shown in FIG. 8D, for example, is taken from a more proximal position and the embedded stiff wall portion 68 takes up significantly more than half of the circumference of the sheath 3. The single gap 70 between ends of the stiff wall portion 68 is about 45 degrees of the circumference forming a C-shaped tube similar to the stiff wall portion 52 described above. Moving distally to the cross-section shown in FIG. 8C shows an additional set of four smaller gaps 70 added to the larger gap. These gaps, as shown in FIG. 8A, tend to define the stiff wall portion 68 into discrete fingers 74. With the increase of the gap size in proportion to the size of the stiff wall portion 68, the expansion stiffness of the sheath 3 drops. The cross-section shown in FIG. 8B is at the distal end and now the stiff wall portion 68 is not present, substantially increasing the expandability of the distal end of the sheath 3.

The gaps 70 can have a range of sizes and positioning, although the gaps shown in FIGS. 8A-8D extend longitudinally and generally parallel to each other. The smaller gaps are circumferentially arranged and spaced from each other and from the larger gap. The multiple gaps 70 with regular spacing facilitate even expansion of elastic tubular layer 66. The full axial length gap can also be of similar circumferential size as the other gaps 70 for a more even distribution of expansion. For example, for six gaps, a 300% strain of a C-shaped tube is divided into 50% at each location. In contrast, tips with a single gap have more localized expansion of the layer 66 and some risk of fracture.

It should be noted that the term 'axial' as used herein is not limited to a straight axis but instead is referring to the general instantaneous direction of a longitudinal structure. In other words, the axis bends with a bend of the elongate structure.

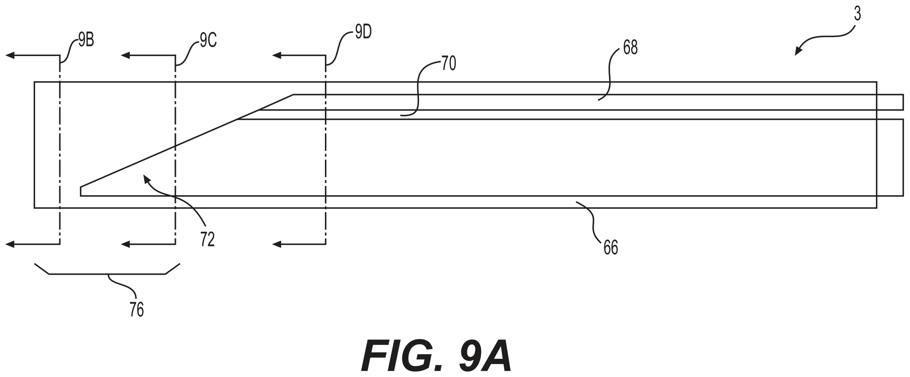

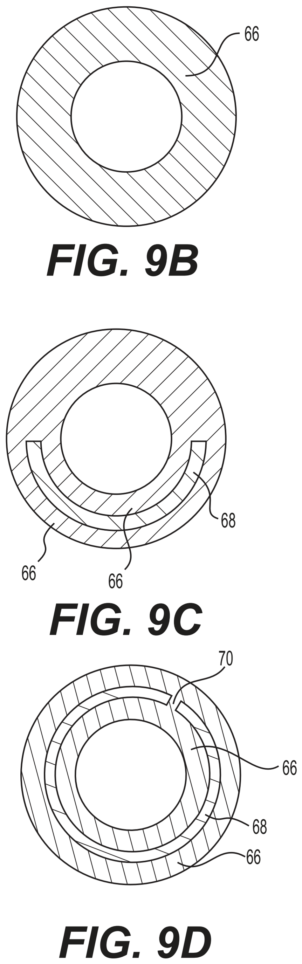

FIGS. 9A-9D show another embodiment wherein the sheath 3 has a single one of the gaps 70 extending longitudinally and then a diagonal cut forming a distal-facing diagonal surface. The diagonal cut serves to progressively decrease the amount of cross-section occupied by the stiff wall portion 68 as it extends in the distal direction, as shown by FIGS. 9D, 9C and 9B.

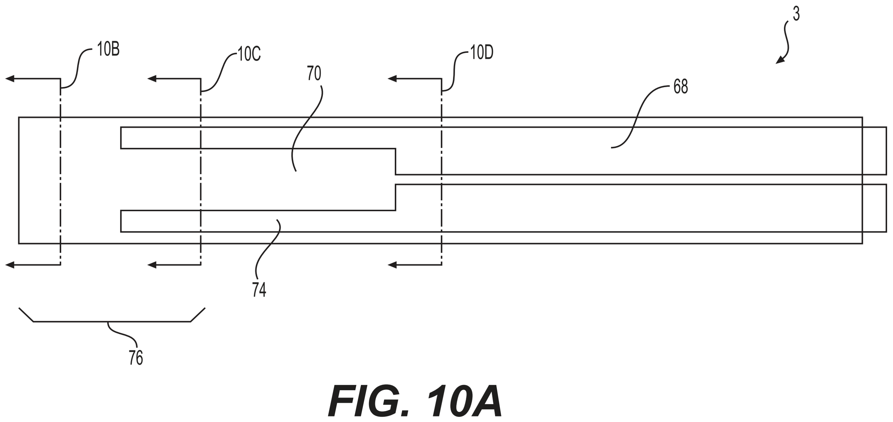

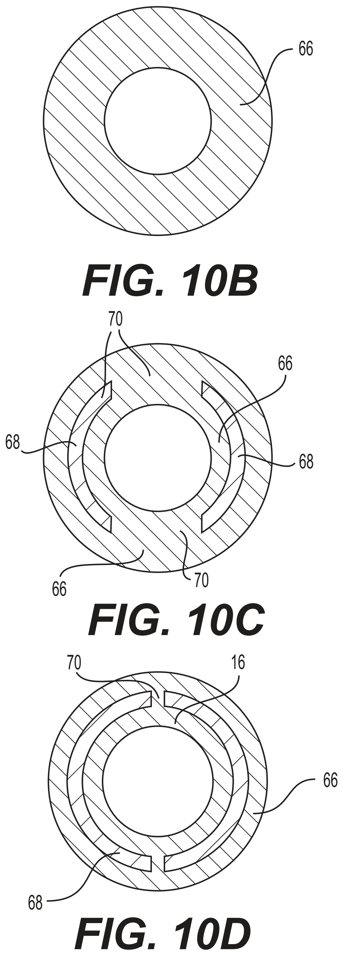

FIGS. 10A-10D show another embodiment wherein the sheath includes a pair of gaps 70 on opposite sides of the stiff wall portion. The pair of gaps expand in the distal direction, being smallest in diameter at the proximal cross-section of FIG. 10D, making a step increase in size to the cross-section of FIG. 10C. At the final transition, the stiff wall portion 68 disappears for cross-section FIG. 10B. This pattern provides a step decrease in resistance to expansion with each transition in the distal direction.

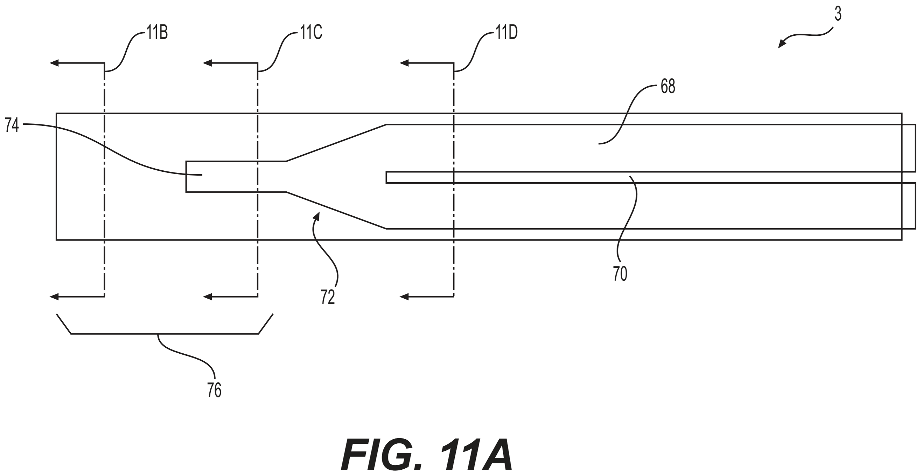

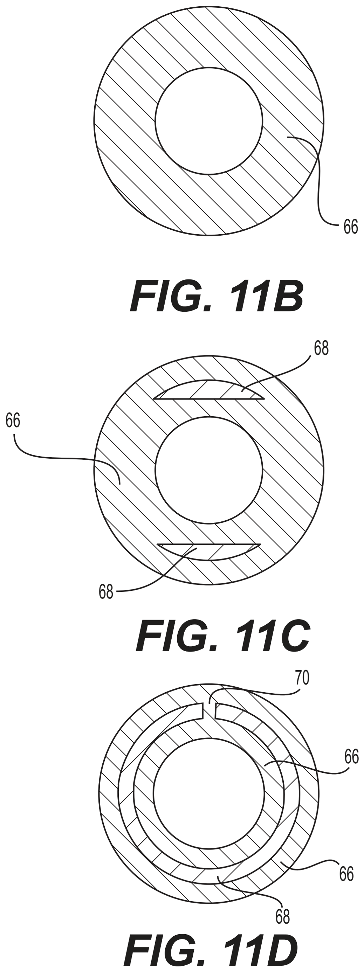

FIGS. 11A-11D show another embodiment wherein one of the gaps 70 disappears when the stiff wall portion starts a pair of converging diagonal surfaces 72. The diagonal surfaces converge to a single pair of opposing fingers 74. Again, the change in proportion of circumference occupied by the stiff wall portion 68 and gaps 70 adjusts the resistance to expansion of the distal end of the sheath 3.

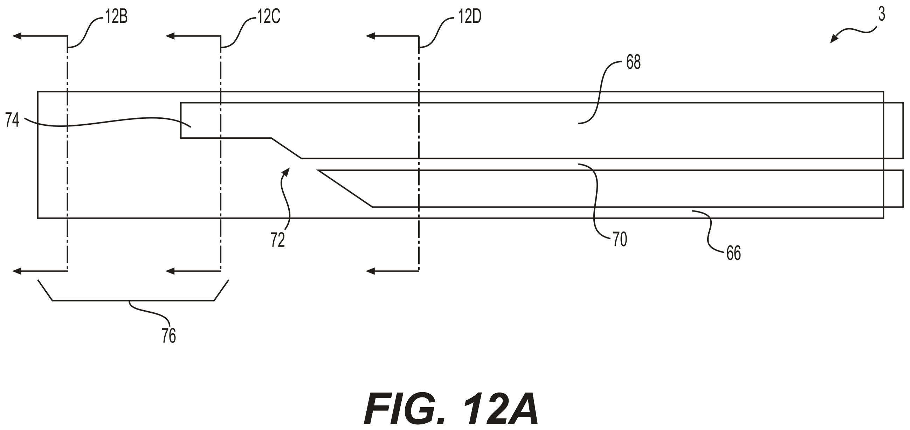

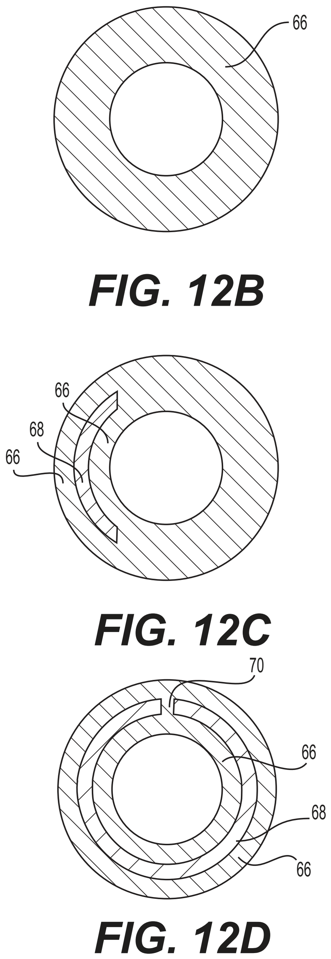

FIGS. 12A-12D show a combination of some of the prior concepts, wherein the sheath 3 includes the diagonal surface 72 converging to one finger 74.

In the embodiments of FIGS. 8A-12D, the elastic tubular layer 66 and stiff wall portion can move independently of one another for freer expansion. This can be supplemented with addition of a tip region 76, such as by reflowing a soft expandable tube or coating over the distal end of the cuts defining the gaps 70 in the C-shaped stiff wall portion 68. Adding the tip can soften and contour the tip for easier insertion of the sheath 3 as well as protect and cover the distal end of the stiff wall portion 68. In FIGS. 8A-8D the tip region 76 covers some or all of the longitudinal length of the fingers 74 while the remainder of the stiff wall portion with only the single C-shaped cross-section (e.g., FIG. 8D cross-section) is left independent of the elastic tubular layer 66 for free expansion. In FIGS. 9A-12D, the tip region can start distal of the termination of the single gap defining the C-shaped cross section of FIG. 9D.

Although embodiments of the sheath 3 disclosed herein have particular layer constructions, they can include additional layers extending around the inside or outside of the layers depicted in the figures. For example, in some implementations, an undercut/bard or tie layer can be included to keep the stiff wall portion 68 attached to the elastic tubular layer 66. In some implementations, a lubricious outermost layer can be included. The lubricious outermost layer can include a slip additive to increase outer surface lubricity.

In some implementations, such as the one shown in FIG. 6B, the first and second layer 54, 62 and wall portion 52 (which is another layer) are bound together, for example, due to fabrication methods that include coextrusion, heat bonding, glue, or another fixative material. Coextruded implementations are particularly advantageous as they are simple and inexpensive to manufacture. Coextrusion also reduces delamination of outer circumferential layers from inner circumferential layers. In other implementations, the layers are not fully bound and are at least partially, and possibly fully, rotatable with respect to each other. For rotatable implementations, the circumferential tension experienced when an implant 5 is passing through is distributed around the layers 20, 54 and 66, instead of being localized to particular locations. This reduces the chance of rupturing those outer layers. In some implementations, the layers are bound together over certain lengths of the sheath 3, and rotatable over other lengths of the sheath 3. In some implementations, the first and second circumferential layers are bound together only at the distal end region of the sheath 3. Selectively allowing rotation of some portions of the layers allows for some improved tear resistance while preserving some element of structural stiffness. In some implementations, the proximal end of sheath 3 can be tapered to attach to external components of the sheath.

In some implementations, various portions of the illustrated embodiments can be supplemented with the longitudinal rods 50. The rods can extend, either partially or fully, along the length of the inner-most surface defining the lumen 32 of the sheath. The longitudinally extending rods can, for example, be supported by the inner-most surface. Here the term "supported by" can mean that the rod is in contact with or extends through that inner surface. For example, the rod can be adhered to or formed on the inner most surface. In some implementations, the longitudinally extending rods can be fully embedded within the inner-most layer. In other implementations, longitudinally extending rods 50 can be partially embedded within the layer, and partially protruding into the inner lumen of the sheath, such as is shown in FIG. 6B.

The height and width of the longitudinally extending rods 50, and thus the amount of the sheath cross-section devoted to the non-elastomeric portions, can vary along the length of sheath 3. A width 43 of the longitudinally extending rods 50 can be, for example, from 0.001 to 0.05 inches. The rods 50 can be circular, ellipsoidal, polygonal, rectangular, square, or a combination of parts of the afore-listed shapes when viewed from a cross section taken generally perpendicular to an elongate axis 2 of the sheath 3. Rods 50 with curved surfaces that protrude into the lumen, such as circular or ellipsoidal surfaces, have the advantage of reducing the area of contact, and therefore the friction, between the sheath and a passing object. Longitudinally extending rods also minimize dimensional change in the longitudinal direction when the sheath is under tension.

Components described as elastic herein can be constructed of elastomers, such as a highly elastic polymer. In some implementations, the elastomeric portion can include polyether, polyurethane, silicone, thermoplastic elastomers, rubber such as styrene-butadiene rubber, or a copolymer of any of the afore-listed highly elastic polymers. The elastomeric material can have an elongation at yield of around 800%. In some implementations, the elastomeric components can comprise a NEUSOFT polymer. The hardness of the NEUSOFT polymer can be, for example, 63 Shore A. NEUSOFT is a translucent polyether urethane based material with good elasticity, vibration dampening, abrasion and tear resistance. The polyurethanes are chemically resistant to hydrolysis and suitable for overmolding on polyolefins, ABS, PC, PEBAX and nylon. The polyuerthane provides a good moisture and oxygen barrier as well as UV stability.

The heightened elasticity of various elastic layers, such as layers 20, 62 and 66, facilitates expansion of the layer from its starting profile to allow for the passage of a prosthetic implant 5 and/or delivery capsule 13. In some implementations, an in particular for passage of a capsule containing a stent-mounted prosthetic implant, the lumen can expand to 0.15-0.4 inches, in a fully expanded state. For example, in one implementation, the original diameter of the lumen is 0.13 inches, expands to 0.34 inches during passage of an implant, and shrinks back to 0.26 inches immediately after passage of the implant and continues to shrink with time until eventually returning back to about 0.13 inches. After the passage of the implant, the lumen collapses back to a narrower diameter due to the elasticity of the elastomeric components.

The non-elastomeric components of embodiments described herein (sometimes particularly described as stiff) are made of a generally stiff material that is less elastic than the elastomeric components. The stiff components lend strength to the sheath 3 to complement the elastic properties contributed by the elastomeric components. The stiffer, non-elastomeric components also contribute to buckle resistance (resistance to failure under pressure), kink resistance (resistance to failure during bending), and torque (or ease of turning the sheath circumferentially within a vessel). The stiff material used to fabricate the stiff components can include high density polyethylene (HDPE), Nylon, polyethylene terephthalate (PET), fluoropolymers (such as polytetrafluoroethylene or PTFE), Polyoxymethylene (POM) or any other suitably stiff polymer. The elongation at yield of the non-elastomeric, stiff components can be, for example, around 5%. The hardness of an HDPE non-elastomeric, stiff component can be, for example, around 70 Shore D.

The non-elastomeric components can also be made of a material that is more lubricious than the elastomeric components, and so as to reduce friction between components and/or the components and the implant 5, capsule 13 or other adjacent contacting objects.

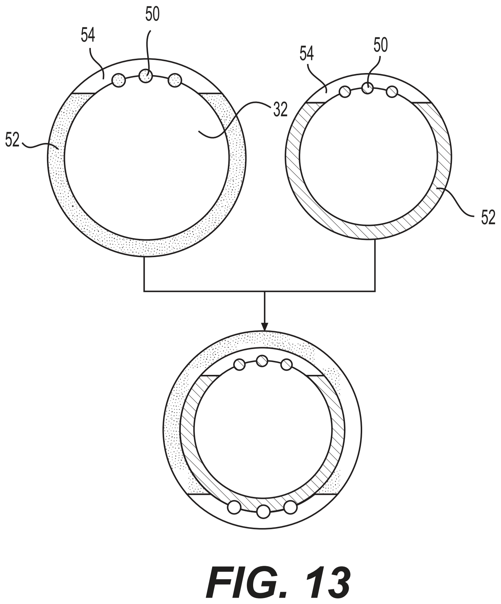

Embodiments disclosed herein can be employed in combinations with each other to create sheaths with varying characteristics. FIG. 13 shows combination of two single-layer tubes nested into each other. Each of the single layer tubes includes a stiff wall portion 52 having a C-shape and an elastic wall portion 54 to close the C-shape around lumen 32. Each single layer tube also includes rods 50 in a similar configuration to the embodiment of FIG. 6B. One of the single layer tubes has a smaller diameter and fits within the lumen 32 of the other tube. The advantage of this combination is a more balanced distribution of elastic wall portions 54 on both sides of the tube which in turns distributes the strains of expansion. The other embodiments disclosed herein can be nested within each other to adjust expansion resistance and distribution.

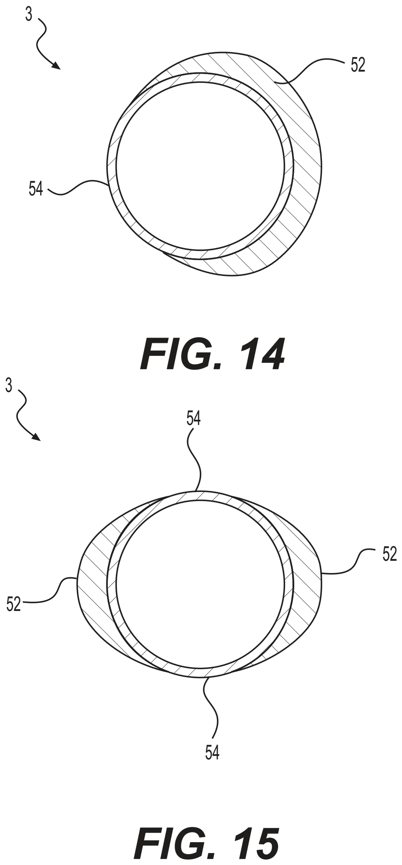

FIGS. 14, 15 and 16 show variations of the sheath 3 that include stiff wall portion 52 and elastic wall portion 54, with the elastic wall portion having a lesser wall thickness for additional flexibility in comparison with the stiff wall portion 52. In these embodiments the wall portions can have the same material with the additional flexibility being due to the reduced thickness. Or the reduced thickness can be combined with more elastomeric material composition.

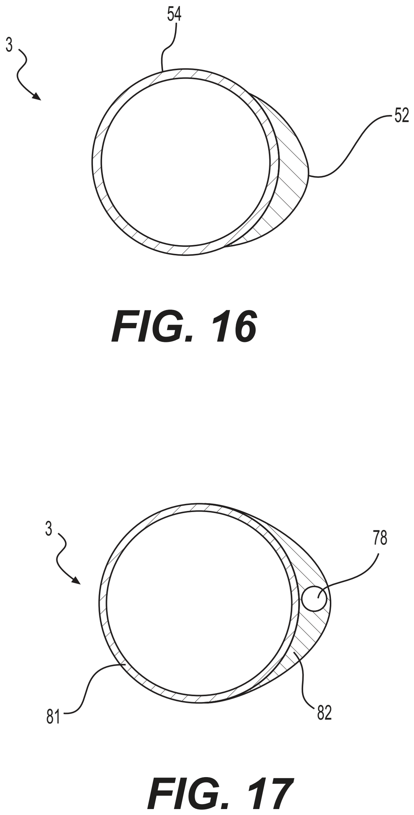

FIG. 14 shows an embodiment of the sheath 3 with a C-shaped stiff wall portion 52 combined with a thin elastic wall portion 54. FIG. 15 shows the use of two elastic wall portions 54 and two thick, stiffer wall portions 52 on opposing sides, positioning the strain of expansion on opposing sides of the sheath 3. FIG. 16 shows an embodiment of the sheath 3 with more than half or 2/3 or 3/4 of the circumference of the sheath being a thinned elastic wall portion 54.

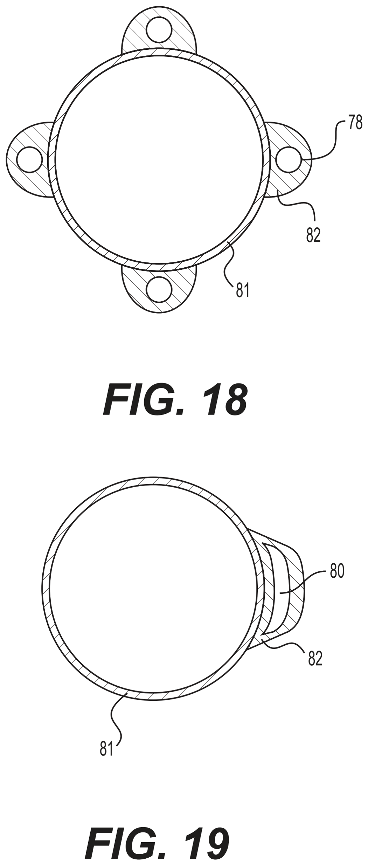

FIGS. 17, 18 and 19 show embodiments wherein wires 78 or strips 80 can be embedded into structures 82 to selectively reinforce an expandable, elastic tubular layer 81. The structures 82 can be thickened mounds or features applied longitudinally - such as be co-extrusion - to the outside surface of the elastic tubular layer 81. The wires or strips can be constructed of relatively stiffer materials for selective reinforcement. FIGS. 17 and 18 show the use of wires 78 and, for increased stiffness, FIG. 19 shows the use of a strip 80 embedded in the structure 82.

The sheaths of FIGS. 14-19 can be manufactured as described above, including via reflowing, gluing, bonding, welding, etc. Materials can include HDPE or TECOFLEX for the stiffer components. Other materials herein can also be used for stiff or elastic components. Also, the materials compositions can be varied to include metals, ceramics and other materials than just polymers. Other features can be applied to the embodiments of FIGS. 14-19 including a lubricious liner, hydrophilic or other coatings, silicone or other liquids or printing.

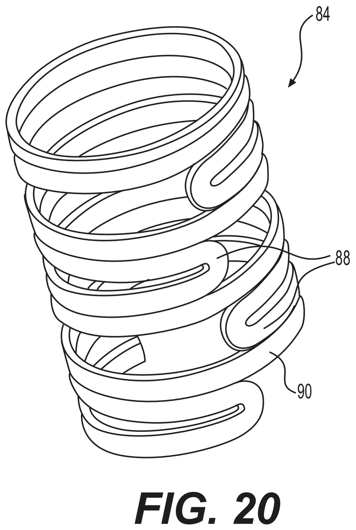

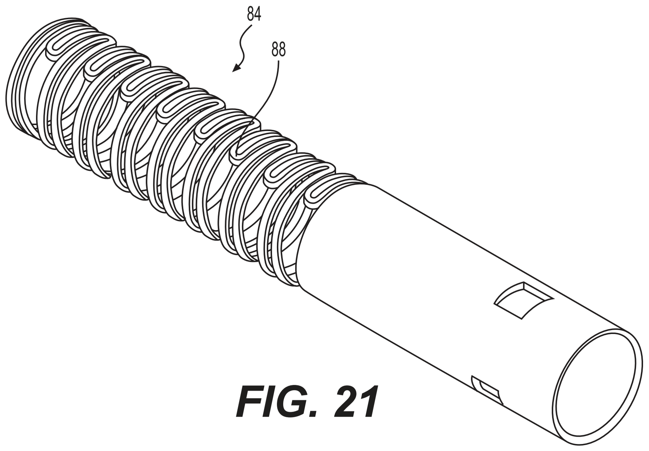

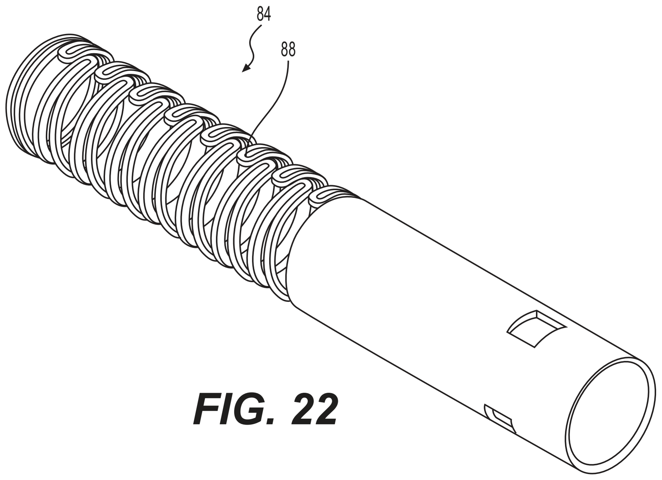

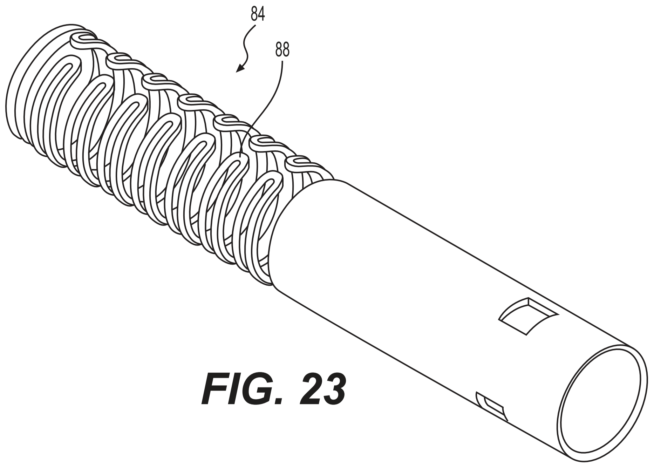

As shown in FIGS. 20-23, another embodiment of the sheath 3 can include a stent structure 84 for embedding in an elastic tubular layer. The stent 84 can include a plurality of loops 88 facing in opposite circumferential directions and that interdigitate between (FIGS. 21-23) or adjacent each other (FIG. 21) so as to be able to open up under pressure of the implant 5 passing therethrough. FIG. 20 shows an additional full circular winding 90 in between each of the loops 88 for additional stiffness. FIGS. 21, 22 and 23 show the progressive expansion of the lumen within the stent 84 as the implant 5 passes therethrough. The stent 84 can have varying lengths and in the illustrated embodiments is used for the distal end of the sheath 3. The stent 84 could also include a heat fused tip on its distal end as shown in other embodiments.

The stent 84 is a shaped frame that can be formed from a laser cut tube or by bending wire into the frame. Similar to the C-shaped stiff tubes, the stent 84 results in an off-center axial load during passage of the prosthetic implant 5. The adjacent relationship of the loops 88 and/or windings 90 provide for excellent pushing stiffness to resist buckling while still having circumferential/radial expandability. Thus, the sheath has a particularly high ratio of buckling to expansion force - allowing for good articulation with easy expansion. The stent 84 is also particularly suited for protecting delicate implants 5, like stent-mounted prosthetic heart valves. The stent 84 can be coated by polymers for hemostatic sealing and protection of the external structures of the prosthetic implant 5.

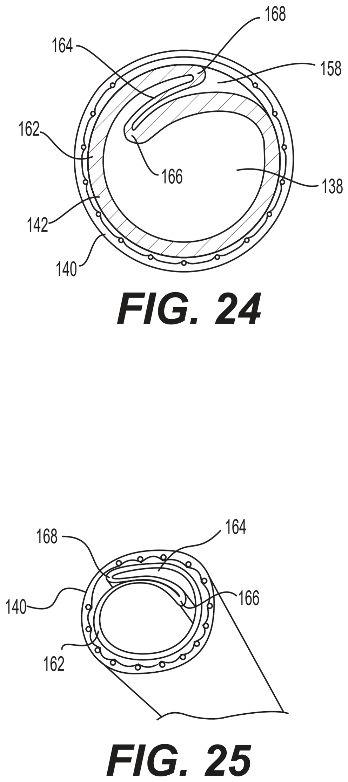

Another embodiment of an introducer sheath is shown in cross section at FIGS. 24 and 25, the sheath having a tubular wall structure including an elastic outer tubular layer 140 and an inner tubular layer 142. The cross-sectional views of FIGS. 24 and 25 show intermediate regions of the sheath (away from proximal or distal ends), in a non-expanded state. In the non-expanded state, a portion of the inner tubular layer 142 is folded over upon itself to fit within the central lumen 158 of the outer tubular layer 140. In some embodiments, the inner and outer tubular layers 142, 140 can be adhered to each other at the distal end of the sheath, in a sealing configuration.

FIG. 26 provides a cross-sectional view taken at an intermediate region along the longitudinal axis of the sheath embodiment of FIGS. 24 and 25, and showing an example inner tubular layer 142 of the sheath in an expanded state. The inner tubular layer 142 can include a thick wall portion 162 integrally connected with a thin wall portion 164. In some embodiments, the thick wall portion 162 can be approximately 0.011 +/- 0.003 inches and the thin wall portion 164 can be approximately 0.0055 +/- 0.0020 inches. The thick wall portion 162, in the illustrated embodiment of FIG. 26, has a C-shaped cross-section with a first longitudinally extending end 166 and a second longitudinally extending end 168. At the ends 166, 168, the thickness of the thick wall portion 162 starts to narrow to thin wall portion 164 on the cross-section. That transition extends longitudinally in the direction of the axis of the sheath, such that the thick wall portion 162 forms an elongate C-shaped member.

The thin wall portion 164 extends between the longitudinally extending ends 166, 168 of the thick wall portion 162 to define the tubular shape of the inner tubular layer 142. As illustrated in FIGS. 24 and 25, in the non-expanded state, the elastic outer tubular layer 140 urges the first longitudinally extending end 166 toward and/or under the second longitudinally extending end 168 of the inner tubular layer 142. This causes the thin wall portion 164 to fold and be positioned between the first and second longitudinally extending ends 166, 168 of the thick wall portion 162. Some embodiments can include multiple folds at various positions around the circumference of the inner tubular layer 142. For example, the inner tubular layer 142 could include two folds spaced 180 degrees from each other, three folds spaced 120 degrees from each other, four folds spaced 90 degrees from each other, and so on.

In some embodiments, the inner tubular layer is stiffer than outer tubular layer. FIG. 28 shows a cross section of an example inner tubular layer 242 in the expanded state, at an intermediate region of the sheath along the longitudinal axis. In some embodiments, the inner tubular layer 265 can be coextruded with multiple segments, as shown in FIG. 28. The different segments can comprise different materials that vary in durometer and coefficient of friction. Optimally, the inner and outer surfaces of the inner tubular layer 242 have a low coefficient of friction to facilitate sliding of the layer against the delivery system, the outer tubular layer, and/or the inner tubular layer itself (to fold and unfold from the expanded state). Advantageously, the thick wall portion 262 can have an overall higher durometer than the thin wall portion 264 to reduce the risk of kinking during insertion. However, the coextrusion process allows for a varying of material type, both of the thick and thin wall portions, to balance the need for high strength and low friction.

The coextrusion processes described herein facilitate fabrication of differently sized sheaths. The coextrusion of the inner tubular layer takes place prior to the folding step. When the inner tubular layer is folded (i.e., the thin wall portion 264 is folded over or under the thick wall portion), the arc length of the thin wall portion 264 helps to determine the inner diameter of sheath. In other words, the coextruded arc length of the thin wall portion 264 must be long enough to allow for a fold that decreases the lumen to the desired size. The coextrusion processes disclosed herein facilitate modifications to the arc length of the thin wall portion 264, making it easy to fabricate sheaths with varying sizes.

FIG. 28 shows an inner tubular layer 242 having a thin wall portion 264 that is coextruded continuously with an innermost segment 265 and an outermost segment 267 of a thick wall portion 262. As such, the radially outermost segment 267 is formed of the same material as the radially innermost segment 265 and the thin wall portion 264 of the inner tubular layer. A radially intermediate layer 269 is coextruded between the radially innermost segment 265 and the radially outermost segment 267 of the thick wall portion 262. The radially intermediate segment 269 can be formed of a stronger material, or a material having a higher durometer, than the radially innermost segment 265 and the radially outermost segment 267 of the thick wall portion 262. Some exemplary strong materials for the radially intermediate segment 269 can include, but are not limited to, polyimide, polyetheretherketone (PEEK), nylon, polyurethane, polyethylene, polyamide, or compounding with fillers and/or combinations. Some exemplary low-friction materials for the thin wall portion 264/radially innermost segment 265/radially outermost segment 267 can include, but are not limited to, high density polyethylene (HDPE), fluoropolymers (such as, but not limited to, fluorinated ethylene propylene or FEP), nylon, polyurethane, polyethylene, polyamide, or compounding and combinations thereof.

As mentioned above, the type and amount of material can be varied to balance the need for high strength and low friction. For example, the arc length of a high durometer, radially intermediate segment 269 of the thick wall portion 262 shown in FIG. 28 could be lengthened to improve kink resistance (the arc length being driven by the folding mandrel and extruded inner diameter). The thickness of the radially intermediate segment 269 could be increased to increase the overall durometer of the thick wall portion 262.

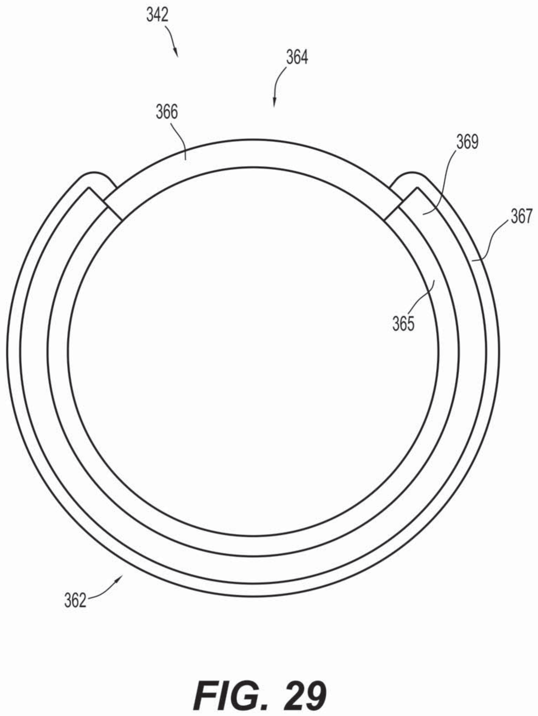

FIG. 29 shows another example of a multisegmented inner tubular layer 342, shown as a cross section taken at an intermediate region along the longitudinal axis of a sheath. Like the embodiment of FIG. 28, the radially inner and outermost segments 365, 367 of the thick wall portion 362 are formed of the same low-friction material, which can include, but is not limited to, HDPE, nylon, polyurethane, polyethylene, polyamide, or compounding with fillers and/or combinations thereof, or fluoropolymer, like FEP. The radially intermediate segment 369 of the thick wall portion 362 can include stronger materials such as, but not limited to, polyimide, PEEK, nylon, polyurethane, polyethylene, polyamide, or compounding with fillers and/or combinations thereof. In FIG. 29, the thin wall portion 364 is formed of a separate coextruded segment 366 that is different from the materials used for the radially innermost, intermediate, and outermost segments of the thick wall portion 362. The material of the thin wall portion segment 366 can be, but is not limited to, soft and low friction materials such as, but not limited to, fluoropolymers such as FEP, nylon, polyurethane, polyethylene, polyamide, polyether block amide (PEBAX), or compounding with fillers and/or combinations thereof. During extrusion, the materials of the various segments are selected to melt together at a desired heat and pressure, creating bonds at the connection regions between the thin wall portion segment 366 and the three radial segments 365, 367, 369 of the thick wall portion 362

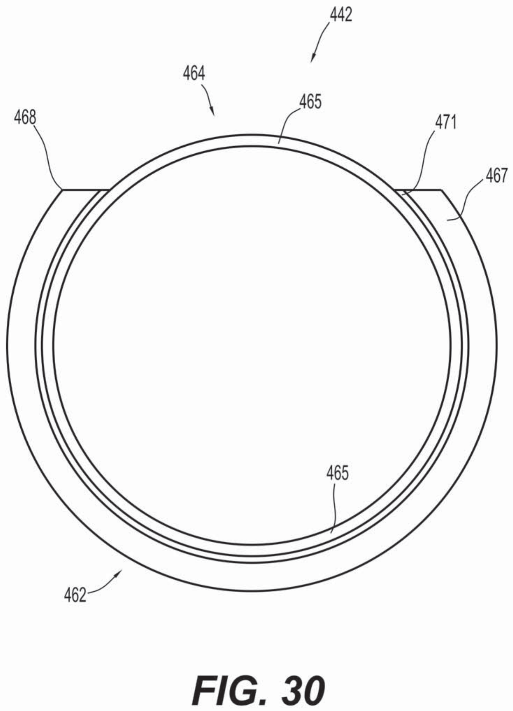

FIG. 30 shows another embodiment of a multisegmented inner tubular layer 442. The inner tubular layer 442 of FIG. 30 includes a thick wall portion 462 and a thin wall portion 464. The thin wall portion 464 is coextruded as a continuous material with the innermost surface of the thick wall portion 462, such that together they make up a first, radially innermost segment 465 of the inner tubular layer 442. The thick wall portion 462 comprises an outermost segment 467 of material, positioned radially outward of the innermost segment 465. The innermost segment 465 can be formed of a low-friction material, including, but not limited to, fluoropolymers such as FEP, or HDPE. The outermost segment 467 can be formed of a strong material, including, but not limited to, polyimide, PEEK, nylon, polyurethane, polyethylene, polyamide, HDPE, or compounding and combinations thereof. Longitudinally extending edges 468 of the outermost segment 467 can be angled as shown, or they can be rounded.

The embodiment of FIG. 30 also includes a tie layer 471 extending between the radially innermost segment 465 and the radially outermost segment 467 around the circumferential length of the thick wall portion 462. The tie layer serves to adhere the material of the innermost segment 465 to the material of the outermost segment 467. The tie layer material acts as a glue layer, bonding during co-extrusion to many dissimilar materials so the material junctions are joined, limiting the chance of delamination. Though tie layer 471 is depicted in FIG. 30 as a radially intermediate segment of the thick wall portion 462, in alternate embodiments tie layers can be used between other configurations of coextruded segments where the materials do not easily adhere to each other. The tie layer material can include, but is not limited to, maleic anhydride modified polyolefins, ethylene vinyl acetate (EVA), and ethylene methyl acrylate (EMA). In some embodiments, tie layer material is a maleic anhydride modified LLDPE.

Multisegmented inner tubular layers not explicitly described herein are within the scope of the disclosure. For example, an innermost segment of the thick wall portion may have a lower coefficient of friction than an outermost segment of the thick wall portion. The number of coextruded segments can vary, and can include 2, 3, 4, 5, 6, 7, 8, 9, 10 or more coextruded segments at a given cross section of the inner tubular layer along the longitudinal axis.

Referring back to FIGS. 24 and 25, generally, the expanded inner diameter of central lumen 138 should approximate the size of the outer diameter of an unexpanded prosthetic device that will pass through it during operation. In an example sheath, the central lumen 138 of the inner tubular layer 142, in the expanded state, has an inner diameter larger than the initial, non-expanded, inner diameter of the central lumen 158 of the elastic outer tubular layer 140. For example, the expanded diameter of the central lumen 138 of the inner tubular layer 142 can be about 0.300 +/- 0.005 inches if intended for use with a crimped valve that has an outer diameter of 0.300 inches. The initial, non-expanded, inner diameter of the central lumen 158 of the outer tubular layer 140 can be about 0.185 inches. In another example for use with a smaller valve, the expanded inner diameter of the central lumen 138 of the inner tubular layer can be about 0.255 +/- 0.005 inches and the initial, non-expanded, inner diameter of the central lumen 158 of the outer tubular layer 140 can be about 0.165 inches +/- 0.005. The elastic outer tubular layer 140 can expand to accommodate an increase in diameter of the inner tubular layer 142 as the prosthetic device passes therethrough.

FIG. 27 taken at an intermediate region along the longitudinal axis of the sheath embodiment of FIGS. 24 and 25, and showing an example outer tubular layer 140. As illustrated, the outer tubular layer 140 has a cylindrical shape with a circular cross-section. The outer tubular layer 140 defines a central lumen 158 extending axially through its cylindrical cross-section. The diameter of the outer tubular layer 140 in its fully expanded state is sized so as to accommodate the implant and its delivery apparatus. In one example sheath, upon expansion, the diameter of the central lumen 158 of the outer tubular layer 140 can be 0.322 inches, the outer tubular layer itself having a wall thickness of 0.005 +/- 0.003 inches to accommodate delivery of a stent-mounted heart valve. In one aspect, inner surface of the outer tubular layer 140 and/or outer surface of the inner tubular layer 142 can be treated to have or have applied thereto a lubricious coating to facilitate unfolding and folding of the inner tubular layer 142.

The central lumen 158 of the outer tubular layer 140 is referred to as having "initial" diameter to designate its passive, non-expanded, or as-formed diameter or cross-sectional dimension when not under the influence of outside forces, such as the implant and its delivery system passing therethrough. In an example sheath, the outer tubular layer 140 can be constructed from an elastic material and may not retain its shape under even light forces such as gravity. Also, the outer tubular layer 140 need not have a cylindrical cross-section and instead could have oval, square or any other regular or irregular shape in cross-section which generally can be configured to meet the requirements of the inner tubular layer 142 and/or expected shape of the implant. Thus, the term "tube" or "tubular" as used herein is not meant to limit shapes to circular cross-sections. Instead, tube or tubular can refer to any elongate structure with a closed-cross section and lumen extending axially therethrough.

The outer tubular layer 140, in one implementation, is constructed of a relatively elastic material having sufficient flexibility to accommodate the expansion induced by passage of the implant and its delivery system and expansion of the inner tubular layer 142 while, at the same time, having enough material strength to urge the inner tubular layer 142 back into/towards a non-expanded state having an approximation of the initial diameter once the implant has passed. In some embodiments, an exemplary material includes NEUSOFT. NEUSOFT is a translucent polyether urethane based material with good elasticity, vibration dampening, abrasion and tear resistance. The polyurethanes are chemically resistant to hydrolysis and suitable for overmolding on polyolefins, ABS, PC, PEBAX and nylon. The polyurethane provides a good moisture and oxygen barrier as well as UV stability. One advantage of the outer tubular layer 140 is that it provides a fluid barrier for the pressurized blood. Other materials having similar properties of elasticity can also be used for the elastic outer tubular layer 140.

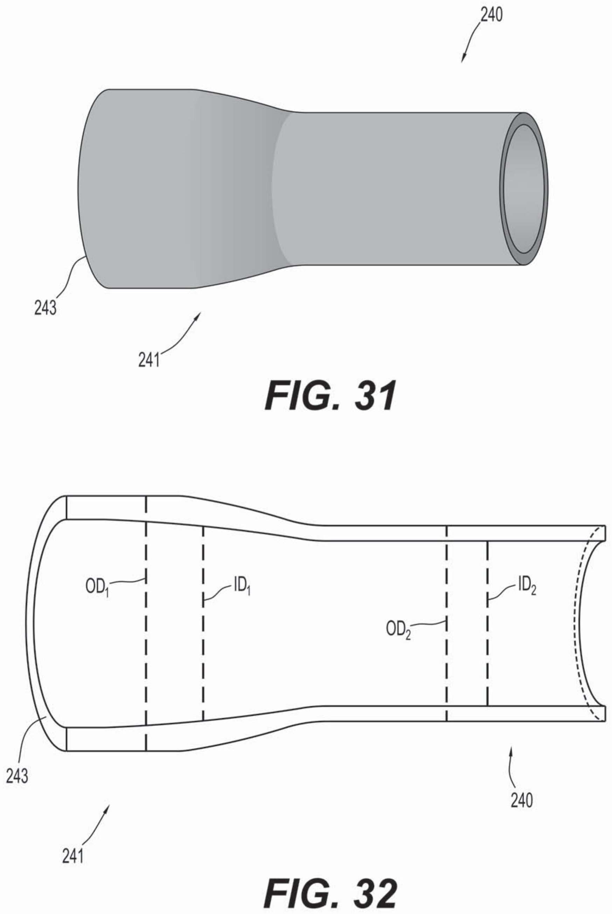

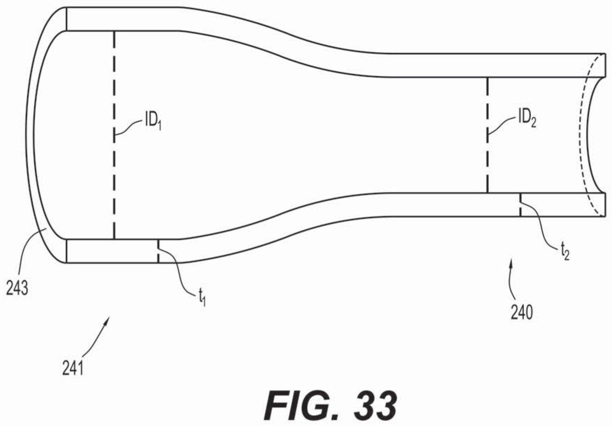

At the proximal end, the sheath widens, or tapers. As mentioned above in reference to FIG. 5, the proximal region can be sized and shaped to accept a distal male end of a hub structure containing, among other things, a hemostasis valve. In some embodiments, the outer diameter **OD** of the outer tubular layer 240 can widen as it approaches the proximal end 243, as shown in FIG. 31. The widening proximal region 241 can extend distally from the proximal end 243 of the outer tubular layer 240 for approximately 3 to 6 inches. As shown in 32, the outer tubular layer 240 can include a proximal region 241 that thickens as it approaches the proximal end 243, such that the **OD** increases in the proximal direction (OD₁ > OD₂) while the inner diameter **ID** is substantially constant, or changes only slightly between ID₁ and ID₂. In some embodiments, the inner diameter of the outer tubular layer 240 varies longitudinally by a value of less than 10%. Alternatively, and as shown in FIG. 33, the wall thickness t of proximal region 241 can stay constant, or change only slightly, between **t₁** and **t₂**, while the inner and outer diameters both change significantly to form widening proximal region 241 (ID₁ > ID₂ and OD₁ > OD₂).

The outer tubular layer 240 of the embodiments of FIGS. 31-33 can be formed of a bump tubing. These configurations are advantageous in that they do not require a seal in the middle of the tapered proximal region to maintain hemostasis. In some embodiments, the widening of the outer tubular layer 240 as it approaches the proximal end 243 can be accomplished by extrusion. In others, the widening of the outer tubular layer 240 can be accomplished by a bonding/reflow operation.

Expandable sheaths of the present disclosure can be used with various methods of introducing a prosthetic device into a patient's vasculature. Generally, during use, the expandable sheath is passed through the skin of patient (usually over a guidewire) such that the distal end region of the expandable sheath is inserted into a vessel, such as a femoral artery, and then advanced to a wider vessel, such as the abdominal aorta. The delivery apparatus and its prosthetic device is then inserted through the expandable sheath and advanced through the patient's vasculature until the prosthetic device is delivered to the implantation site and implanted within the patient. During the advance of the prosthetic device through the expandable sheath, the device and its delivery system exerts a radially outwardly directed force. Referring back to the embodiment shown in FIGS. 24 and 25, the radially outwardly directed force will be exerted on a portion of the inner tubular layer 142, and that portion of the inner tubular layer 142 exerts a corresponding radially outwardly directed force on a portion of the outer tubular layer 140, causing both the inner tubular layer 142 and the outer tubular layer 140 to expand locally to accommodate the profile of the device. The expansion of the inner tubular layer 142 causes the first and second longitudinally extending ends 166, 168 of the thick wall portion 162 to radially expand/separate. As a result, the thin wall portion 164 unfolds from its contracted state to define the expanded diameter of the inner tubular layer 142.

As the prosthetic device and its delivery system passes through the expandable sheath, the expandable sheath recovers. That is, it returns to its original, non-expanded configuration. This is facilitated by outer tubular layer 140, which has a higher elastic modulus than inner tubular layer 142. The outer tubular layer can provide an inwardly directed radial force to exert a compressive force urging the inner tubular layer 142 towards the non-expanded state. The outer tubular layer 140 can urge the first and second longitudinally extending ends 166, 168 toward and/or under, each other, after the passage of the prosthetic implant, such that the ends 166, and 168 of the inner tubular member 142 overlap when in the non-expanded state, with the thin wall portion 164 extending therebetween.

As described above, the expandable sheath can be used to deliver, remove, repair, and/or replace a prosthetic device. In one example, the expandable sheath described above can be used to deliver a prosthetic heart valve to a patient. For example, a heart valve (in a crimped or compressed state) can be placed on the distal end portion of an elongated delivery catheter and inserted into the sheath. Next, the delivery catheter and heart valve can be advanced through the patient's vasculature to the treatment site, where the valve is implanted.

Beyond transcatheter heart valves, the expandable sheath can be useful for other types of minimally invasive surgery, such as any surgery requiring introduction of an apparatus into a subject's vessel. For example, the expandable sheath can be used to introduce other types of delivery apparatus for placing various types of intraluminal devices (e.g., stents, stented grafts, balloon catheters for angioplasty procedures, valvuloplasty procedures, etc.) into many types of vascular and non-vascular body lumens (e.g., veins, arteries, esophagus, ducts of the biliary tree, intestine, urethra, fallopian tube, other endocrine or exocrine ducts, etc.).

In view of the many possible embodiments to which the principles of the disclosed invention can be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

The invention further comprises the following embodiments:
1. An expandable sheath comprising: an elastic outer tubular layer; and a multisegmented inner tubular layer comprising at least two coextruded segments, the at least two coextruded segments having different durometers and different coefficients of friction, the inner tubular layer further comprising a thick wall portion integrally connected to a thin wall portion, the thick wall portion having a first and second longitudinally extending end, the thin wall portion extending between the first and second longitudinally extending ends, wherein the thin wall portion has a lower durometer than the thick wall portion, wherein the elastic outer tubular layer and the inner tubular layer are radially movable between an expanded state and a non-expanded state, wherein in the non-expanded state the elastic outer tubular layer urges the first longitudinally extending end under the second longitudinally extending end of the inner tubular layer, wherein in the expanded state the first and second longitudinally extending ends of the inner tubular layer expand apart with the thin wall portion extending circumferentially therebetween, and wherein the outer elastic tubular layer urges the inner tubular layer towards the non-expanded state.
2. The expandable sheath of embodiment 1, wherein the at least two coextruded segments have different arc lengths extending in the circumferential direction.
3. The expandable sheath of either embodiment 1 or embodiment 2, wherein the durometer of the inner tubular layer varies radially through the thick wall portion.
4. The expandable sheath of any one of the above embodiments, wherein the coefficient of friction of the inner tubular layer varies radially through the thick wall portion.
5. The expandable sheath of any one of the above embodiments, wherein a radially outermost segment of the thick wall portion is formed of the same material as a radially innermost segment of the thick wall portion.
6. The expandable sheath of any one of the above embodiments, wherein the material of the radially outermost segment and the radially innermost segment of the thick wall portion is HDPE.
7. The expandable sheath of any one of the above embodiments, wherein a radially intermediate segment of the thick wall portion has a higher durometer than the radially outermost segment and the radially innermost segment.
8. The expandable sheath of any one of the above embodiments, wherein the radially intermediate segment of the thick wall portion is C-shaped in cross section and has an arc length that is less than the full arc length of the thick wall portion.
9. The expandable sheath of any one of the above embodiments, wherein the radially outermost segment and the radially innermost segment meet at longitudinally extending edges of the radially intermediate segment to fully envelop the radially intermediate segment.
10. The expandable sheath of any one of the above embodiments, wherein the material of the thin wall portion is continuous with the material of the radially innermost segment and the radially outermost segment of the thick wall portion.
11. The expandable sheath of any one of the above embodiments, wherein the thin wall portion comprises a first coextruded material and the thick wall portion comprises the first coextruded material and a second coextruded material positioned radially outward from the first coextruded material.
12. The expandable sheath of embodiment 11, wherein the first coextruded material forms a radially innermost segment of the inner tubular layer and the second coextruded material forms a radially outermost segment of the inner tubular layer.
13. The expandable sheath of embodiment 11, wherein the first coextruded material has a lower coefficient of friction than the second coextruded material.
14. The expandable sheath of any one of embodiments 1-9, wherein the material of the thin wall portion has a lower durometer than the material of the radially innermost segment and the radially outermost segment of the thick wall portion.
15. The expandable sheath of any one of embodiments 1-9 or 14, wherein the thin wall portion is formed of a different coextruded segment than the radially innermost segment, the radially intermediate segment, and the radially outermost segment of the thick wall portion.
16. The expandable sheath of any one of embodiments 1-15, further comprising a coextruded tie layer, wherein the coextruded tie layer serves to adhere a first coextruded segment to a second coextruded segment.
17. The expandable sheath of embodiment 16, wherein the tie layer adheres a radially innermost segment of the inner tubular layer to a radially outermost segment of the inner tubular layer.
18. The expandable sheath of any one of embodiments 1-17, wherein the at least two coextruded segments extend a portion of the length of the multisegmented inner tubular layer.
19. The expandable sheath of any one of embodiments 1-17, wherein the at least two coextruded segments extend the full length of the multisegmented inner tubular layer.
20. The expandable sheath of any one of embodiments 1-19, wherein the thick wall portion makes up greater than 50% of the circumference of a wall of the inner tubular layer.
21. The expandable sheath of any one of embodiments 1-20, wherein the inner tubular layer comprises a fold in the unexpanded state.
22. The expandable sheath of any one of embodiments 1-21, wherein the outer tubular layer further comprises a tapered proximal end, and the outer tubular layer widens nearing the proximal end of the sheath.
23. The expandable sheath of any one of embodiments 1-22, wherein the outer diameter of the outer tubular layer increases nearing the proximal end of the sheath while the inner diameter of the outer tubular layer changes by a value of less than 10% nearing the proximal end of the sheath.
24. The expandable sheath of any one of embodiments 1-23, wherein the outer diameter of the outer tubular layer increases nearing the proximal end of the sheath, the inner diameter of the outer tubular layer increases nearing the proximal end of the sheath, and the thickness of the outer tubular layer changes by a value of less than 10% nearing the proximal end of the sheath.
25. The expandable sheath of any one of embodiments 1-24, wherein the tapered proximal end widens in a gradual manner to create a curved outer surface.
26. The expandable sheath of any one of embodiments 1-25, wherein the elastic outer tubular layer comprises at least one longitudinally extending reinforcement formed of a higher durometer material than the material immediately adjacent to the reinforcement.
27. The expandable sheath of any one of embodiments 1-26, wherein the elastic outer tubular layer is seamless and prevents fluid leakage.
28. The expandable sheath of any one of embodiments 1-27, wherein at least one surface of the sheath comprises a hydrophilic coating.
29. The expandable sheath of any one of embodiments 1-28, wherein the sheath is sized to accommodate the delivery of a heart valve.
30. The expandable sheath of any one of embodiments 1-29, wherein the outer diameter of the outer tubular layer is from 0.22 inches to 0.30 inches.
31. The expandable sheath of any one of embodiments 1-30, wherein the configuration of the inner tubular layer changes moving longitudinally such that a distal tip of the expandable sheath has a distinct configuration as compared to a longitudinally central shaft of the expandable sheath.
32. The delivery catheter assembly of any one of embodiments 1-31, wherein the expandable sheath is an introducer sheath.
33. A delivery catheter assembly comprising; a proximal region comprising a hub and a hemostasis valve, the expandable sheath of any one of embodiments 1-32, the expandable sheath coupled to and extending distally from the hub and fluidically coupled to the hemostasis valve, a guide catheter slidably positionable within the expandable sheath, a balloon catheter positionable within the guide catheter, a distal region of the balloon catheter comprising an inflatable balloon, an implantable device configured to be coupled to the inflatable balloon, and a capsule configured to extend over the implantable device.
34. The delivery catheter assembly of embodiment 33, wherein the implantable device is a heart valve.
35. The delivery catheter assembly of either of embodiments 33 or 34, wherein the guide catheter is steerable.
36. The delivery catheter assembly of any one of embodiments 33-35, further comprising a nose cone at the distal region of the balloon catheter.
37. The delivery catheter assembly of any one of embodiments 33-36, wherein a tapered proximal end of the sheath widens as it extends toward the proximal hub.
38. The delivery catheter assembly of any one of embodiments 33-37, wherein the tapered proximal end of the sheath is coupled to the hub.
39. The delivery catheter assembly of any one of embodiments 33-38, wherein the hemostasis valve is housed within the hub.
40. The delivery catheter assembly of any one of embodiments 33-39, wherein the proximal region of the delivery catheter assembly further comprises a handle.
41. The delivery catheter assembly of any one of embodiments 33-40, wherein the handle further comprises an infusion port.
42. A method of inserting an implantable device into the blood vessel of a patient, the method comprising: providing the expandable sheath or delivery catheter assembly of any one of embodiments 1-41; inserting the expandable sheath at least partially into the blood vessel of the patient; advancing an implantable device through the inner tubular layer of the sheath;locally expanding the inner tubular layer from the compressed condition to the locally expanded condition using the outwardly directed radial force of the implant; and locally contracting the inner tubular layer from the locally expanded condition at least partially back to the compressed condition using inwardly directed radial force of the outer elastic tubular layer.
43. The method of embodiment 42, wherein locally expanding further comprises moving the first and second longitudinally extending ends towards and then away from each other to reach the locally expanded condition.
44. The method of either of embodiments 42 or 43, wherein locally contracting further comprises moving the first and second longitudinally extending ends toward and then away from each other to at least partially reach the compressed condition.
45. The method of any one of embodiments 42-44, wherein advancing an implantable device further comprises sliding a guide catheter through the expandable sheath with an implantable device coupled thereon.
46. A method of positioning an implantable device within the vasculature of a patient, the method comprising: performing the method of any one of embodiments 42-45; advancing the implantable device distally beyond a distal tip of the sheath; positioning the implantable device within the vasculature of the patient; and removing the sheath from the blood vessel of the patient.
47. The method of embodiment 46, further comprising expanding the implantable device within the vasculature of the patient.
48. The method of embodiment 47, wherein expanding the implantable device within the vasculature of the patient further comprises expanding a heart valve within the vasculature of the patient.
49. The method of either of embodiments 47 or 48, wherein expanding the implantable device within the vasculature of the patient further comprises inflating a balloon to apply a radially outward force on an inner surface of the implant.
50. The method of any of embodiments 46-49, further comprising removing a capsule from the outer surface of the implantable device.

## Claims

1. An expandable sheath comprising:
an elastic outer tubular layer (140); and
a multisegmented inner tubular layer (442) comprising at least two coextruded segments, the at least two coextruded segments having different durometers and different coefficients of friction, the inner tubular layer (442) further comprising a thick wall portion (462) integrally connected to a thin wall portion (464), the thick wall portion (462) having a first and second longitudinally extending end, the thin wall portion (464) extending between the first and second longitudinally extending ends,
wherein the thin wall portion (464) has a lower durometer than the thick wall portion (462),
wherein the elastic outer tubular layer (140) and the inner tubular layer (442) are radially movable between an expanded state and a non-expanded state,
wherein in the non-expanded state the elastic outer tubular layer (140) urges the first longitudinally extending end under the second longitudinally extending end of the inner tubular layer (442),
wherein in the expanded state the first and second longitudinally extending ends of the inner tubular layer (442) expand apart with the thin wall portion (464) extending circumferentially therebetween, and
wherein the outer elastic tubular layer urges the inner tubular layer (442) towards the non-expanded state, and
wherein the thin wall portion (464) comprises a first coextruded material (465) and the thick wall portion (462) comprises the first coextruded material (465) and a second coextruded material (467) positioned radially outward from the first coextruded material (465).

2. The expandable sheath of claim 1, wherein the at least two coextruded segments have different arc lengths extending in the circumferential direction;
preferably wherein the durometer of the inner tubular layer (442) varies radially through the thick wall portion (462).

3. The expandable sheath of claim 1, wherein the coefficient of friction of the inner tubular layer (442) varies radially through the thick wall portion (462).

4. The expandable sheath of any one of the above claims, wherein a radially outermost segment of the thick wall portion (462) is formed of the same material as a radially innermost segment of the thick wall portion (462);
preferably wherein the material of the radially outermost segment and the radially innermost segment of the thick wall portion (462) is HDPE.

5. The expandable sheath of any one of the above claims, wherein a radially intermediate segment of the thick wall portion (462) has a higher durometer than the radially outermost segment and the radially innermost segment.

6. The expandable sheath of any one of the above claims, wherein the radially intermediate segment of the thick wall portion (462) is C-shaped in cross section and has an arc length that is less than the full arc length of the thick wall portion (462).

7. The expandable sheath of any one of the above claims, wherein the radially outermost segment and the radially innermost segment meet at longitudinally extending edges of the radially intermediate segment to fully envelop the radially intermediate segment.

8. The expandable sheath of any one of the above claims, wherein the material of the thin wall portion (464) is continuous with the material of the radially innermost segment and the radially outermost segment of the thick wall portion (462).

9. The expandable sheath of claim 1, wherein the first coextruded material (465) forms a radially innermost segment of the inner tubular layer (442) and the second coextruded material (467) forms a radially outermost segment of the inner tubular layer (442); and/or
wherein the first coextruded material (465) has a lower coefficient of friction than the second coextruded material (467).

10. The expandable sheath of any one of the above claims, further comprising a coextruded tie layer, wherein the coextruded tie layer serves to adhere a first coextruded segment to a second coextruded segment;
preferably wherein the tie layer adheres a radially innermost segment of the inner tubular layer (442) to a radially outermost segment of the inner tubular layer (442).

11. The expandable sheath of any one of claims 1-10, wherein the at least two coextruded segments extend a portion of the length of the multisegmented inner tubular layer (442).

12. The expandable sheath of any one of claims 1-10, wherein the at least two coextruded segments extend the full length of the multisegmented inner tubular layer (442).

13. The expandable sheath of any one of the above claims, wherein the thick wall portion (462) makes up greater than 50% of the circumference of a wall of the inner tubular layer (442).

14. The expandable sheath of any one of the above claims, wherein the inner tubular layer (442) comprises a fold in the unexpanded state.

15. The expandable sheath of any one of the above claims, wherein the elastic outer tubular layer (140) comprises at least one longitudinally extending reinforcement formed of a higher durometer material than the material immediately adjacent to the reinforcement.
